# EUROPEAN PATENT SPECIFICATION

(11) **EP 2 279 262 B1**
(45) Date of publication and mention of the grant of the patent: **25.12.2013**
(21) Application number: 09734926.0
(22) Date of filing: 24.04.2009
(51) Int. Cl.: C12Q 1/48

(54) **MEASUREMENT OF PROTEIN KINASE ACTIVITY IN CEREBROSPINAL FLUID FOR DIAGNOSIS OF NEUROLOGICAL AND PSYCHIATRIC DISORDERS**
MESSUNG VON PROTEINKINASE-AKTIVITÄT IN LIQUOR ZUR DIAGNOSE NEUROLOGISCHER UND PSYCHIATRISCHER STÖRUNGEN
MESURE DE L'ACTIVITÉ DE LA PROTÉINE KINASE DANS LE FLUIDE CÉPHALO-RACHIDIEN POUR LE DIAGNOSTIC DE TROUBLES NEUROLOGIQUES ET PSYCHIATRIQUES

(30) Priority: 25.04.2008 EP 08155211
(43) Date of publication of application: 02.02.2011
(73) Proprietor: PamGene B.V., 5211 's Hertogenbosch (NL); Vereniging voor Christelijk Hoger Onderwijs, Wetenschappelijk Onderzoek en Patiëntenzorg, 1081 HV Amsterdam (NL)
(72) Inventor: HILHORST, Maria, Helena, NL-6706 CP Wageningen (NL); DE WIJN, Richard, NL-6542 PS Nijmegen (NL); HOOZEMANS, Jeroen, Joseph, Maria, NL-1061 BK Amsterdam (NL); VAN DER VIES, Saskia, Maria, NL-1185 CC Amstelveen (NL)
(74) Representative: De Clercq, Ann G. Y.
(86) International application number: PCT/EP2009/054994
(87) International publication number: WO 2009/130320

(56) References cited:
- WO-A-2007/054326
- WO-A-2008/030579
- US-A- 4 874 694
- US-A1- 2007 082 366
- RUIJTENBEEK ROB: "Peptide microarrays in protein kinase and nuclear receptor research" INTERNET CITATION, [Online] XP002420886 Retrieved from the Internet: URL:http://www.ngpharma.com/pastissue/arti cle.asp?art=268321&issue=170> [retrieved on 2007-02-08]
- BEUNINGEN VAN R ET AL: "Kinase profiling in a new dimension on PamChip(R) arrays" INTERNET CITATION, [Online] XP002385619 Retrieved from the Internet: URL:http://www.pamgene.com/content/pdf/rin ie_kinase.pdf> [retrieved on 2006-06-16]
- RONQUIST G ET AL: "Cerebrospinal fluid markers of disturbed brain cell metabolism." PROGRESS IN NEUROBIOLOGY 1982, vol. 18, no. 2-3, 1982, pages 167-180, XP002493679 ISSN: 0301-0082
- RONQUIST G ET AL: "Adenylate kinase activity and glutathione concentration of cerebrospinal fluid in different neurological disorders" EUROPEAN NEUROLOGY, XX, SWITZERLAND, vol. 18, no. 2, 1 January 1979 (1979-01-01), pages 106-110, XP008095762 ISSN: 0014-3022
- SCHUTKOWSKI MIKE ET AL: "Peptide arrays for kinase profiling." CHEMBIOCHEM : A EUROPEAN JOURNAL OF CHEMICAL BIOLOGY MAR 2005, vol. 6, no. 3, March 2005 (2005-03), pages 513-521, XP002493680 ISSN: 1439-4227
- CHAPEL HELEN ET AL: "Shigella sonnei meningitis due to interleukin-1 receptor-associated kinase-4 deficiency: first association with a primary immune deficiency." CLINICAL INFECTIOUS DISEASES : AN OFFICIAL PUBLICATION OF THE INFECTIOUS DISEASES SOCIETY OF AMERICA 1 MAY 2005, vol. a40, no. 9, 1 May 2005 (2005-05-01), pages 1227-1231, XP002538913 ISSN: 1537-6591
- KLAFKI HANS-WOLFGANG ET AL: "Measurement of ERK 1/2 in CSF from patients with neuropsychiatric disorders and evidence for the presence of the activated form.", JOURNAL OF ALZHEIMER'S DISEASE : JAD 2009, vol. 18, no. 3, 2009, pages 613-622, ISSN: 1875-8908
- KUMAR SATHISH ET AL: "Extracellular phosphorylation of the amyloid [beta]-peptide promotes formation of toxic aggregates during the pathogenesis of Alzheimer's disease.", THE EMBO JOURNAL 1 JUN 2011, vol. 30, no. 11, 1 June 2011 (2011-06-01), pages 2255-2265, ISSN: 1460-2075
- ISHIKAWA HIROYUKI O ET AL: "The Raine syndrome protein FAM20C is a Golgi kinase that phosphorylates bio-mineralization proteins.", PLOS ONE 2012, vol. 7, no. 8, 2012, page e42988, ISSN: 1932-6203
- SCHUTZER STEVEN E ET AL: "Establishing the proteome of normal human cerebrospinal fluid.", PLOS ONE 2010, vol. 5, no. 6, 2010, page e10980, ISSN: 1932-6203

## Description

### Field of the invention

The present invention relates to the use of cerebrospinal fluid for the classification, diagnosis and prognosis of neurological and psychiatric disorders as well as for predicting and monitoring treatment effects. More specifically it relates to the use of endogenous protein kinase activity in cerebrospinal fluid for the above purpose. In particular an array of substrates for protein kinases, immobilized on a porous matrix, is used to monitor the protein kinase activity in cerebrospinal fluid.

### Background

Cerebrospinal fluid (CSF) is a clear bodily fluid that occupies the subarachnoid space and the ventricular system around and inside the brain. More specifically CSF occupies the space between the arachnoid mater and the pia mater. Moreover it constitutes the content of all intra-cerebral ventricles, cisterns and sulci, as well as the central canal of the spinal cord. CSF is an approximately isotonic solution that acts as a buffer for the cortex, providing also a basic mechanical and immunological protection to the brain inside the skull. CSF is usually obtained by a lumbar puncture. The CSF contains approximately 0.3% plasma proteins depending on sampling site.

Different characteristics of CSF, such as the protein, glucose and cellular content, are known to be used in medicine for the diagnosis of a variety of neurological diseases. These parameters alone may be extremely beneficial in the diagnosis of subarachnoid haemorrhage and central nervous system infections, such as meningitis. Using more sophisticated methods for analysis of CSF, e.g. the detection of the oligoclonal bands, an ongoing inflammatory condition, such as multiple sclerosis, can be recognized.

The protein composition of cerebrospinal fluid is largely derived from serum proteins which leak in to the subarachnoid space through imperfections in the blood brain barrier, such as the area postrema, and perhaps across the choroid plexus, the richly vascular structure through which cerebrospinal fluid is generated as an ultrafiltrate. Some proteins, such as immunogloblulins may be generated in the subarachnoid space during inflammation. Since the cerebrospinal fluid bathes the surfaces of cerebral and cerebellar cortices, the caudate, brainstem and spinal cord, some contribution of these structures to total cerebrospinal fluid protein might be expected.

The protein composition of CSF has been reported to change as consequence of certain neurological disorders. It may to a certain extend reflect what is happening in the neurons. The occurrence of certain proteins in CSF has been related to Creutzfeldt-Jakob disease, Schizophrenia and Alzheimer's disease. The presence of single proteins or combinations of proteins obtained through 2D-electrophoresis or ELISA, have been used to classify these disorders.

Signal transduction refers to any process by which a cell converts one kind of signal or stimulus to another and is one of the most important biological processes that is currently under investigation. Signal transduction involves an ordered sequence of biochemical reactions, which are carried out by enzymes that activate secondary messengers. Through this process cells regulate various activities needed for life. The regulation of signal transduction processes involves changes in protein phosphorylation. As many as up to 1000 kinases, more than 500 protein kinases and 500 phosphatases in the human genome are thought to be involved in phosphorylation processes. The targets of phosphorylation encompass a large group of signalling molecules, including enzymes.

It has already been established that protein kinases, both tyrosine, serine and threonine kinases, play an important role in signalling pathways that are known to play key roles in various diseases. However only a limited number of the known protein kinases have been investigated so far. The present invention therefore provides a method for monitoring the activity of protein kinases. The inventors of the present invention have shown that CSF surprisingly contains active protein kinases and that the differences in protein kinase activity can be linked to various neurological and/or psychiatric disorders.

The method of the present invention provides a convenient diagnostic tool to use in the diagnosis of disorders. Many such disorders, especially the neurodegenerative disorders have heretofore been diagnosed by exclusion and were based on clinical criteria supported by neuropsychological tests and neuroimaging. Especially in the early stage of the disorder it is difficult to distinguish different types of dementia, since the clinical symptoms are often subtle. Hence a significant number of diagnoses cannot be made or turn out to be wrong based on analysis of post-mortem brain material. Therefore there is a need for new methods and systems for the diagnosis of neurodegenerative disorders. Therefore, new techniques that enable the diagnosis and discrimination between neurodegenerative disorders are warranted. Obviously, a simpler, less-invasive technique would be a welcome addition to the diagnostic arts. This invention provides such a diagnostic tool which utilizes only a small sample of the patients' cerebrospinal fluid.

WO2007054326 discloses a method for measuring protein kinase activity in brain biopsy samples. The method comprises incubating the sample with a microarray upon which peptide substrates for protein kinases are immobilized.

### Summary of the invention

In its broadest sense the invention pertains to subject-matter as defined in the appended claims.

Cerebrospinal fluid is known in medicine as a source for the diagnosis of a variety of neurological diseases. The present application relates to the use of cerebrospinal fluid (CSF) for the classification, diagnosis, prognosis and prediction of treatment effects. More specifically the inventors have shown that, notwithstanding the low protein content in CSF compared to other bodily fluids, the method of the present invention enables monitoring protein kinase activity in cerebrospinal fluid and detecting the effect of a pharmacologic compound on this activity. Furthermore, different neuronal disorders are reflected in the protein kinase activity.

Accordingly, within one embodiment of the present invention, a method is provided for analyzing CSF and determining the presence or development of a pathology, which can be neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease. The method comprises the steps of:
a) providing a sample of cerebrospinal fluid;
b) incubating said sample with ATP on an array of substrates; and,
c) obtaining a detectable phosphorylation profile, said profile resulting from the interaction of the cerebrospinal fluid sample with the array of substrates.

More preferably the substrates used in the present invention are peptide substrates for protein kinases and the array of substrates is a flow-through array.

In another embodiment, the present invention relates to the use of a method according to the invention, for diagnosis of neurological and psychiatric disorders.

### Description of the figures

**FIG. 1** shows the results of an incubation of CSF samples on a PamChip®.
**FIG. 2** shows analysis of the phosphorylation profiles differentiating between 3 classes of CSF samples having different pathologies
**FIG. 3** shows the principal component analysis of the phosphorylation patterns obtained with CSF samples having different pathologies.
**FIG.4** provides, as depicted in the examples, a graphical representation where IL-1β induced IL-6 secretion by U373 astrocytoma cells and human primary astrocytes is reduced by IRAK1/4 inhibitor.

### Detailed description

Before the present method and devices used in the invention are described, it is to be understood that this invention is not limited to particular methods, components, or devices described, as such methods, components, and devices may, of course, vary. It is also to be understood that the terminology used herein is not intended to be limiting.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The present application relates to the use of cerebrospinal fluid (CSF) for the classification, diagnosis, prognosis and prediction of treatment effects. More specifically the inventors have shown that, notwithstanding the low protein content in CSF, the method of the present invention enables monitoring protein kinase activity, and preferably endogenous protein kinase activity, in cerebrospinal fluid. The inventors surprisingly found that clinical and post mortem CSF both fresh and frozen contains active protein kinases. It was shown that the protein kinases in CSF are still able to phosphorylate other proteins or peptides. Furthermore, the inventors have surprisingly found that the profiles obtained with CSF from different sources are different. The profiles obtained for one specific disease are reproducible for a given disease state and specific profiles can be obtained for use in diagnosing patients whose particular pathology is unknown or otherwise unconfirmed. The inventors have therefore shown that neuronal disorders are reflected in a differential protein kinase activity which enables an early diagnosis of neurological and/or psychiatric disorders. The variable, disorder related, presence of protein kinases and their ability to phosphorylate specific peptides enables the analysis of CSF of a patient and identification of a certain pathology. More particularly, the present invention relates to a method for analyzing CSF and determining the presence or development of a pathology, which can be neurological diseases, psychiatric disorders, oncological diseases, metabolic diseases, immunological and auto immunological diseases, diseases of the nervous system and/or infectious diseases, preferably neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease.

By 'enzymes' we refer to proteins that are able to catalyze reactions wherein they convert substrates to products without themselves being part of the end products of the reaction. Monomers, oligomers or polymers composed of amino acids, nucleotides or sugars may be modified by enzymes. For protein kinases, the modified substrate might be either another enzyme or any other protein participating in the same signal transduction pathway. Enzymes that may be analyzed include, but are not limited to, oxidoreductases including dehydrogenases, reductases and oxidases; transferases including methyltransferases, carbamoyltransferases, transketolases, acetyltransferases, phosphorylases, phosphoribosyltransferases, sialyltransferase; phosphotransferases including kinases such as calcium/calmodulin dependent kinases, cyclin-dependent kinases, lipid signaling kinases, mitogen-activated protein kinases, PDK1-PKB/Akt, PKA, PKC, PKG, GSK-3beta, non-receptor protein tyrosine kinases, receptor protein tyrosine kinases, serine/threonine kinases, histidine kinases, hydrolases including lipases, esterases, hydrolases, protein phosphatases, phosphodiesterases, glucosidases, galactosidases, amidases, deaminases and pyrophosphatases; lyases including decarboxylases, aldolases, hydratases and ferrochelatases; isomerases including epimerases, isomerases, and mutases; ligases including GMP synthase, CTP synthase, NAD+ synthetase, and carboxylases. The methods according to the present invention are equally directed to enzymes without a known biologically active function.

Accordingly, in the present disclosure, methods are provided wherein the enzymatic activity is chosen from the group comprising kinase activity, phosphatase activity, protease activity, transferase, activity, and proteinase activity. In a more preferred embodiment of the present disclosure, methods are provided wherein the enzymatic activity is kinase activity. In the invention, the enzymatic activity is protein kinase activity.

Kinases are a class of enzymes that transfer phosphate groups from high energy phosphate donors like ATP, GTP, CTP or UTP to a phosphate acceptor molecule. The phosphate acceptors may be small molecules like carbohydrates (e.g. glucose), nucleic acids (e.g. adenylate), lipids, or large molecules like proteins. Adenylate kinase (also known as ADK or myokinase) is an example of such a small molecule kinase. It is a phosphotransferase that catalyzes the interconversion of adenine nucleotides (2 AD ↔ ATP + AMP) and plays an important role in cellular energy homeostasis.

Protein kinases are a special class of kinases. Protein kinase activity is referred to as the activity of protein kinases. A protein kinase is a generic name for all enzymes that transfer a phosphate to a protein. About three to four percent of the human genome contains transcription information for the formation of protein kinases. Currently, there are about 518 known different protein kinases. However, because three to four percent of the human genome is a code for the formation of protein kinases, there may be many more separate kinases in the human body. A protein kinase is an enzyme that modifies proteins by covalently coupling phosphate groups to them. This process or activity is also referred to as phosphorylation. Phosphorylation can therefore be regarded as the process of the addition of a phosphate group to a substrate. Phosphorylation usually results in a functional change of the substrate by changing enzyme activity, cellular location, or association with other proteins. Up to 30% of all proteins may be modified by protein kinase activity, and kinases are known to regulate the majority of cellular pathways, especially those involved in signal transduction, the transmission of signals within the cell. The chemical activity of a protein kinase involves removing a phosphate group from ATP or GTP, or any other phosphate source, and covalently attaching it to amino acids such as serine, threonine, tyrosine, histidine, aspartic acid and/or glutamic acid that have a free hydroxyl group. Most known protein kinases act on both serine and threonine, others act on tyrosine, and a number acts on serine, threonine and tyrosine. The protein kinase activity monitored with the method of the present invention is preferably directed to protein kinases acting towards serine, threonine and/or tyrosine, preferably acting on both serine and threonine, on tyrosine or on serine, threonine and tyrosine and more preferably the method of the present invention if preferably directed to protein kinases acting towards serine and threonine.

Because protein kinases have profound effects on a cell, their activity is highly regulated. Kinases are turned on or off by for instance phosphorylation, by binding of activator proteins or inhibitor proteins, or small molecules, or by controlling their own location in the cell relative to their substrates. Deregulated protein kinase activity is a frequent cause of disease since protein kinases regulate many aspects that control for instance cell growth, movement and death.

Accordingly, within one embodiment of the present invention, a method is provided for analyzing CSF and determining the presence or development of a pathology, which can be neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease. The method comprises the steps of:
a) providing a sample of cerebrospinal fluid;
b) incubating said sample with ATP on an array of substrates; and,
c) obtaining a detectable phosphorylation profile, said profile resulting from the interaction of the cerebrospinal fluid sample with the array of substrates.

The method of the present invention enables the analysis of CSF and determining the activity of protein kinases in a highly sensitive and fast way. It should be noted that the method of the present invention measures the activity of protein kinases in CSF and therefore the method of the present invention measures the endogenous protein kinase activity.

The substrates as used herein, are meant to include proteins, hormone receptors, enzymes and peptides. In particular the substrates used are substrates for protein kinases, more in particular peptide substrates for protein kinases, even more particular the peptide substrates for protein kinases in Table 1, Table 2 and/or Table 3, most particularly using at least 2, 3, 4, 5, 9, 10, 12, 16, 20, 30, 40, 50, 60, 70, 80, 90, 100, 110, 120, 130 or 140 peptides of the peptide substrates for protein kinases in Table 1. In a preferred embodiment the array of substrates comprises at least two peptides selected from the group consisting of the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52. In a more preferred embodiment the array of substrates comprises or consists of the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52.

It should be noted that a person skilled in the art will appreciate that the kinase substrates used in the methods of the present invention and immobilized on the arrays of the invention may be the peptides as listed in Table 1, Table 2 and/or Table 3. These peptides can be used according to the methods or arrays of the present invention to measure the phosphorylation levels of phosphorylation sites of said peptides in the presence of protein kinase present in the samples. The phosphorylation levels of the individual phosphorylation sites present in said peptides may be measured and compared in different ways. Therefore the present invention is not limited to the use of peptides identical to any of the peptides as listed in Table 1, Table 2 and/or Table 3 as such. The skilled person may easily on the basis of the sequence of the peptides listed in Table 1, Table 2 and/or Table 3 design variants compared to the specific peptides in said tables and use such variants in a method for measuring phosphorylation levels of phosphorylation sites present in said peptides as listed in Table 1, Table 2 and/or Table 3. These variants may be peptides which have a one or more (2, 3, 4, 5, 6, 7, etc.) amino acids more or less than the given peptides and may also have amino acid substitutions (preferably conservative amino acid substitutions) as long as these variant peptides retain at least one or more of the phosphorylation sites of said original peptides as listed in said tables. Further the skilled person may also easily carry out the methods or construct arrays according to the present invention by using proteins (full length or Nor C-terminally truncated) comprising the amino acid regions of the peptides listed in Table 1, Table 2 and/or Table 3 as sources for studying the phosphorylation of sites present in the amino acid regions of the peptides listed in Table 1, Table 2 and/or Table 3. Also the skilled person may use peptide mimetics which mimick the peptides listed in Table 1, Table 2 and/or Table 3. The present invention also includes the use of analogs and combinations of these peptides for use in the method or arrays according to the present invention. The peptide analogs include peptides which show a sequence identity of more than 70%, preferably more than 80% and more preferably more than 90%.

As used herein "peptide" refers to a short truncated protein generally consisting of 2 to 100, preferably 2 to 30, more preferably 5 to 30 and even more preferably 13 to 18 naturally occurring or synthetic amino acids which can also be further modified including covalently linking the peptide bonds of the alpha carboxyl group of a first amino acid and the alpha amino group of a second amino acid by eliminating a molecule of water. The amino acids can be either those naturally occurring amino acids or chemically synthesized variants of such amino acids or modified forms of these amino acids which can be altered from their basic chemical structure by addition of other chemical groups which can be found to be covalently attached to them in naturally occurring compounds.

As used herein "protein" refers to a polypeptide made of amino acids arranged in a linear chain and joined together by peptide bonds between the carboxyl and amino groups of adjacent amino acid residues.

As used herein "peptide mimetics" refers to organic compounds which are structurally similar to peptides and similar to the peptide sequences list in Table 1, Table 2 and/or Table 3. The peptide mimetics are typically designed from existing peptides to alter the molecules characteristics. Improved characteristics can involve, for example improved stability such as resistance to enzymatic degradation, or enhanced biological activity, improved affinity by restricted preferred conformations and ease of synthesis. Structural modifications in the peptidomimetic in comparison to a peptide, can involve backbone modifications as well as side chain modification.

**Table 1: list of 140 peptides used for determining the kinase activity, their sequence and Seq.Id.No.**

| | Name | Sequence |
|---|---|---|
| 1 | ABL1_729_740_T729/S733/T735 | TEWRSVTLPRDL |
| 2 | ACM1_421_433_T428 | CNKAFRDTFRLLL |
| 3 | ACM1_444_456_T455/S451 | KIPKRPGSVHRTP |
| 4 | ACM4_456_468_T459/T463 | CNATFKKTFRHLL |
| 5 | ACM5_494_506 T501/T505/Y495 | CYALCNRTFRKTF |
| 6 | ACMS_498_510_T501/T505 | CNRTFRKTFKMLL |
| 7 | ADDB_696_708_S697/S699/S701/S703 | GSPSKSPSKKKKK |
| 8 | ADDB_706_718 T711/S713/S718 | KKKFRTPSFLKKS |
| 9 | ADRB2_338_350_S345/S346/Y350 | ELLCLRRSSLKAY |
| 10 | AKT1_301_313_T305/T308/T312 | KDGATMKTFCGTP |
| 11 | ANXA1_208_220_T215 | AGERRKGTDVNVF |
| 12 | ANXA2_16_28_T18/S17/S21/S25/Y23 | HSTPPSAYGSVKA |
| 13 | ATF2_47-59_T51/T53/T55 | VADQTPTPTRFLK |
| 14 | BCKD_45_57_T49/T51/S47/S52/S57/Y54 | ERSKTVTSFYNQS |
| 15 | CAC1C_1974_1986_S1975/S1981 | ASLGRRASFHLEC |
| 16 | CACD2_494_506_T501/T505 | LEDIKRLTPRFTL |
| 17 | CALD1_723_735_T724/S730 | KTPDGNKSPAPKP |
| 18 | CALD1_746_758_T751/T753 | INEWLTKTPDGNK |
| 19 | CBL_693_705_Y700 | EGEEDTEYMTPSS |
| 20 | CDC2_154_169_Y160/T161/T166/Y169 | GIPIRVYTHEVVTLWY |
| 21 | CDK7_163_175_T170/T175/S164/Y169 | GSPNRAYTHQVVT |
| 22 | CENPA_1_14_S7 | MGPRRRSRKPEAPR |
| 23 | CENP-C_725_737_T734/S732 | HHKLVLPSNTPNV |
| 24 | CFS1R_701_713_S713/Y708 | NIHLEKKYVRRDS |
| 25 | CFTR_730_742_S737/S742 | EPLERRLSLVPDS |
| 26 | CFTR_761_773_S768 | LQARRRQSVLNLM |
| 27 | CFTR_783_795_T787/T788/T791/5790/5795 | IHRKTTASTRKVS |
| 28 | CGHB_109_122_T117/T118/S116 | QCALCRRSTTDCG |
| 29 | CHK2_377_389_T378/T383/T387/T389/S379 | ETSLMRTLCGTPT |
| 30 | COF1_16_28_T24/S22/S23 | DMKVRKSSTPEEV |
| 31 | CREB1_126_138_S129/S133/Y134 | EILSRRPSYRKIL |
| 32 | CSK21_355_367_T360/S356/S357/S362 | ISSVPTPSPLGPL |
| 33 | DCX_49_61_T56/S57 | HFDERDKTSRNMR |
| 34 | ELK1_329_341_T336/S339/S341 | GGPGPERTPGSGS |
| 35 | ELK1_356_368_T359/T361/T363/T368 | LLPTHTLTPVLLT |
| 36 | ELK1_410_422_T417/S411/S416/S422 | ISVDGLSTPWLS |
| 37 | EPB42_240_252_S247 | LLNKRRGSVPILR |
| 38 | ERBB2_679_691_T686/Y685 | QQKIRKYTMRRLL |
| 39 | ERF_519_531_T526/S531 | GEAGGPLTPRRVS |
| 40 | ESR1_160_172_T168/S167 | GGRERLASTNDKG |
| 41 | F263_454_466_T463/S461 | NPLMRRNSVTPLA |
| 42 | F264_436_448_S443/S444 | LNVAAVNTHRDRP |
| 43 | FIBA_569_581_S572/S576/S577/S578/S580/Y579 | EFPSRGKSSSYSK |
| 44 | FOX03_25_37_T32/S26/S30 | QSRPRSCTWPLQR |
| 45 | GPR6_349_361_S350/S356/S358/S360 | QSKVPFRSRSPSE |
| 46 | GPSM2_394_406_S401 | PKLGRRHSMENME |
| 47 | GRB2_427_439_T439/S427/S434 | SRLRRRASQLKIT |
| 48 | GRIK1_718_730_T730/S725/S726 | EKMWAFMSSRQQT |
| 49 | GRIK2_708_720 S710/S711/S715/S720 | FMSSRRQSVLVKS |
| 50 | GSUB_61_73_T68 | KKPRRKDTPALHI |
| 51 | GYS2_1_14_T10/S6/S8/S11 | MLRGRSLSVTSLG |
| 52 | H2B1B_26_39_S32/S36/S38 | GKKRKRSRKESYSI |
| 53 | H2BR_26_38_S32/S36 | DGKKRKRSRKESY |
| 54 | H32_2_17_T3/T6/S10/T11 | RTKQTARKSTGGKAPR |
| 55 | HS90B_218_230_S225 | KEREKEISDDEAE |
| 56 | INSR_1368_1380_T1375/S1379 | KKNGRILTLPRSN |
| 57 | IPP1_28_40_T35/T38 | QIRRRRPTPATLV |
| 58 | K6PL_765_777_T769/T772/S774 | LEHVTRRTLSMDK |
| 59 | KAP2 91_103_T103/S91/S98 | SRFNRRVSVCAET |
| 60 | KAP3_106_118_T109/S113 | NRFTRRASVCAEA |
| 61 | KAPCG_191_205 T195/T197/T201/Y204 | VKGRTWTLCGTPEYL |
| 62 | KCC1A_170_182_T177/T181/S173/S176 | DPGSVLSTACGTP |
| 63 | KCC2G_278_289_S280/T287 | VASMMHRQETVE |
| 64 | KCNA1_438_450_T448/S439/S442/S445/S446/S447/S450 | DSDLSRRSSSTMS |
| 65 | KCNA2_442_454_T452/S447/S449/S451/S454 | PDLKKSRSASTIS |
| 66 | KCNA3_461_473_T471/S468/S470/S473 | EELRKARSNSTLS |
| 67 | KCNA6_504_516_T515/S511/Y512 | ANRERRPSYLPTP |
| 68 | KCNB1_489_501_T491/T494/T498/S496/S499/S500/S501 | KWTKRTLSETSSS |
| 69 | KIF11_920_932_T924/T926/T927/S932 | LDIPTGTTPQRKS |
| 70 | KPB1_1011_1024_S1018/S1020/S1023 | QVEFRRLSISAES |
| 71 | KPCB_18_30_S24_A24S | RFARKGSLRQKNV |
| 72 | KPCB_625_638_T633 | AENFDRFFTRHPPV |
| 73 | LA_359_371_T362/S366 | GKKTKFASDDEHD |
| 74 | LAM1_15_27_T18/T19/T24/S22/S27 | GGPTTPLSPTRLS |
| 75 | LMNA_192_204_T199 | DAENRLQTMKEEL |
| 76 | MARCS_151_163_S158/S162 | KKKKKRFSFKKSF |
| 77 | MARCS_159_171_S162/S166/S169 | FKKSFKLSGFSFK |
| 78 | MBP_222_234_T229/T232 | HFFKNIVTPRTPP |
| 79 | MBP_225_238_T229/T232/S236 | KNIVTPRTPPPSQ |
| 80 | MK10_216_228_T218/T223/T228/S219/Y225 | AGTSFMMTPYVVT |
| 81 | MP2K1_280_292_T285/T291 | GDAAETPPRPRTP |
| 82 | MP2K1_286_298_T291/S297/S298 | PPRPRTPGRPLSS |
| 83 | MPH6_140_152_T147 | EDENGDITPIKAK |
| 84 | MPIP3_208_220_S209/Y212/5214/S216 | RSGLYRSPSMPEN |
| 85 | MYBB_513_525_T515/T518/T520 | DNTPHTPTPFKNA |
| 86 | MYC_51_63_T58/S62 | KKFELLPTPPLSP |
| 87 | MYPC3_268_280_T274/S269/S275 | LSAFRRTSLAGGG |
| 88 | NCF1_296_308_S303/S304 | RGAPPRRSSIRNA |
| 89 | NCF1_321_333_S328/Y324 | QDAYRRNSVRFLQ |
| 90 | NCF1_372_384_T382/S379/S381 | DLILNRCSESTKR |
| 91 | NEK2_171_184_T174/T178/S183/Y180/Y181 | FAKTFVGTPYYM S |
| 92 | NEK3_158_170_T161/T165/Y162/Y167/Y168 | FACTYVGTPYYVP |
| 93 | NFKB1_330_342_T341/S337/S342 | FVQLRRKSDLETS |
| 94 | NMDZ1_890_902_S890/S896/S897/T900/S901/T902 | SFKRRRSSKDTST |
| 95 | NR4A1_344_356_S351 | GRRGRLPSKPKQP |
| 96 | NTRK3_824 836 T831/Y834 | LHALGKATPIYLD |
| 97 | P2AB_297_309_T304/Y307 | EPHVTRRTPDYFL |
| 98 | P53_308_323_T312/S313/S314/S315 | LPNNTSSSPQPKKKPL |
| 99 | PDE5A_95_107_T96/T98/S102/S104 | GTPTRKISASEFD |
| 100 | PHS1_7_19_S14 | QEKRRQISIRGIV |
| 101 | PLEC1_4635_4647_T4646/S4636/S4638/S4642 | RSGSRRGSFDATG |
| 102 | PLEK_106_118 T114/S113/S117 | GQKFARKSTRRSI |
| 103 | PLM_76_88_T79/S82/S83/S88 | EEGTFRSSIRRLS |
| 104 | PPLA_9_21_T17/S10/S16 | RSAIRRASTIEMP |
| 105 | PRGR_786_798_S793 | EQRMKESSFYSLC |
| 106 | PTK6_436_448_T445/S442/S443/S446/Y447 | ALRERLSSFTSYE |
| 107 | PTN12_32_44_T40/T44/S39/Y42 | FMRLRRLSTKYRT |
| 108 | Q5HY18_106_111_S110 | GLRRWSLGGLRRWSL |
| 109 | Q6ICU1_105_118_S106G_S110 | EGLRSRSTRMSTVS |
| 110 | RAB1A_186_198 T194/S187/5193 | KSNVKIQSTPVKQ |
| 111 | RADI_559_569_Y562/T564 | RDKYKTLRQIR |
| 112 | RAF1_252_264_T258/T260/S252/S257/S259 | SQRQRSTSTPNVH |
| 113 | RAP1B_172_184_S179/S180 | PGKARKKSSCQLL |
| 114 | RB_242_254_T252/S249 | AVIPINGSPRTPR |
| 115 | RB_804_816_S807/S811 | IYISPLKSPYKIS |
| 116 | RBL2_410_422_T417/T421/S413/S420 | KENSPCVTPVSTA |
| 117 | RBL2_632_644_T642/S639 | DEICIAGSPLTPR |
| 118 | RBL2_635_637_T642/T647/S639 | CIAGSPLTPRRVT |
| 119 | RBL2_655_667_S662 | GLGRSITSPTTLY |
| 120 | RBL2_687_699_T694/S688/S690 | DSPSDGGTPGRMP |
| 121 | RBL2_955_967_T961/S962/S965/S966 | ELNKDRTSRDSSP |
| 122 | RBL2_959_971_T961/S962/S965/S966/S971 | DRTSRDSSPVMRS |
| 123 | REL_260_272_S267/S272 | KMQLRRPSDQEVS |
| 124 | RS6_228_240_S235/S236/S240 | IAKRRRLSSLRAS |
| 125 | RYR1_4317_4329_T4324 | VRRLRRLTAREAA |
| 126 | SCN7A_898_910_S905/S906 | KNGCRRGSSLGQI |
| 127 | SRC_412_424_T419/Y418 | LIEDNEYTARQGA |
| 128 | SRC8_CHICK_423_435_Y430 | KTPSSPVYQDAVS |
| 129 | STK6_283_295_S283/S284/T287/T288/T292/Y295 | SSRRTTLCGTLDY |
| 130 | STMN2_90_102_S97 | AAGERRKSQEAQV |
| 131 | TAU_523_535_T528/T533/S524/S526/S530 | GSRSRTPSLPTPP |
| 132 | TLE_242_254_T248/T249/S245 | EPPSPATTPCGKV |
| 133 | TNR7_212_224_S219/S224/Y217 | HQRRKYRSNKGES |
| 134 | TY3H_63_77_S70 | RFIGRRQSLIEDARK |
| 135 | VASP_149_161_S156 | EHIERRVSNAGGP |
| 136 | VASP_231_243_S238 | GAKLRKVSKQEEA |
| 137 | VASP_270_282_T277 | LARRRKATQVGEK |
| 138 | VIGLN_287_299_T293/T294/T295 | EEKKKKTTTIAVE |
| 139 | VTNC_390_402_T400/S393/S397 | NQNSRRPSRATWL |
| 140 | ZAP70_486_498_T493/S490/S496/Y491/Y492 | LGADDSYYTARSA |

The term 'cerebrospinal fluid' or 'CSF' as used herein, refers to the fluid that surrounds the bulk of the central nervous system, as described in Physiological Basis of Medical Practice (J. B. West, ed., Williams and Wilkins, Baltimore, Md. 1985). CSF includes ventricular CSF and spinal cord CSF.

The term 'phosphorylation profile' or 'kinase activity profile' as used herein, refers to the response of the substrates, preferably kinase substrates generated during the incubation of at least two such substrates with a CSF sample.

The inventors have found that a particular enzymatic activity such as for instance a kinase activity can be monitored in samples of CSF. This is due to the protein kinases present in CSF. Therefore, contacting the CSF samples with an array of two or more substrates and preferably kinase substrates, and more in particular peptide substrates for protein kinases, in the presence of ATP will lead to a phosphorylation of the kinase substrates.

Alternatively, the phosphorylation of the substrates and/or peptide substrates for protein kinases can be performed in the absence of exogenous ATP. When no ATP is added during the incubation of CSF on the array of substrates, the endogenous ATP, the ATP naturally present in the CSF, will act as the prime source of ATP.

This response of the kinase substrates, also referred to as the phosphorylation profile or kinase activity profile of the sample, can be determined using a detectable signal. The signal is the result from the interaction of the sample with the array of substrates more specifically with the peptide substrates on this array that have been modified by the incubation with the sample. The response of the array of substrates can be monitored using any method known in the art. The response of the array of substrates is determined using a detectable signal, said signal resulting from the interaction of the sample with the array of substrates. As mentioned hereinbefore, in determining the interaction of the sample with the array of substrates the signal is either the result of a change in a physical or chemical property of the detectably labeled substrates, or indirectly the result of the interaction of the substrates with a detectably labeled molecule capable of binding to the substrates or the result of a chemical reaction of a detectable compound with the modified substrate (e.g. Pro-Q Diamond phosphoprotein stain). For the latter, the molecule that specifically binds to the modified peptide substrates of interest (e.g., antibody or polynucleotide probe) can be detectably labeled by virtue of containing an atom (e.g., radionuclide), molecule (e.g. fluorescein), or complex that, due to a physical or chemical property, indicates the presence of the molecule. A molecule may also be detectably labeled when it is covalently bound to or otherwise associated with a "reporter" molecule (e.g., a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labeled avidin or streptavidin conjugate, magnetic beads (e.g., Dynabeads'), fluorescent dyes (e.g., fluorescein, fluorescein-isothiocyanate (FITC), Texas red, rhodamine, green fluorescent protein and all variants known in the art, enhanced green fluorescent protein, lissamine, phycoerythrin, Cy2, Cy3, Cy3.5, Cy5, Cy5.5, Cy7, FluorX [Amersham], SYBR Green I & II [Molecular Probes], Alexa dyes and the like), radiolabels, enzymes (e.g., hydrolases, particularly phosphatases such as alkaline phosphatase, esterases and glycosidases, or oxidoreductases, particularly peroxidases such as horse radish peroxidase, and the like), substrates, cofactors, inhibitors, chemilluminescent groups, chromogenic agents, and colorimetric labels such as colloidal gold or colored glass or plastic (e. g., polystyrene, polypropylene, latex, etc.) beads.

Means of detecting such labels are well known to those of skill in the art. Thus, for example, chemiluminescent and radioactive labels may be detected using photographic film or scintillation counters, and fluorescent markers may be detected using a photodetector to detect emitted light (e.g., a CCD camera, or a method as in fluorescence-activated cell sorting). Enzymatic labels are typically detected by providing the enzyme with a substrate and detecting a colored reaction product produced by the action of the enzyme on the substrate. Preferentially a compound that precipitates upon conversion is used. Colorimetric labels are detected by simply visualizing the colored label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the color associated with the label. Fluorescence resonance energy transfer has been adapted to detect binding of unlabeled ligands, which may be useful on arrays.

In a particular embodiment of the present invention the response of the array of substrates to the sample is determined using detectably labeled antibodies; more in particular antibodies directly or indirectly labeled with a fluorescent label. In those embodiments of the invention where the substrates consist of kinase substrates, the response of the protein kinase substrates is determined using fluorescently labelled anti-phosphotyrosine antibodies, fluorescently labelled anti-phosphoserine or fluorescently labelled anti-phosphothreonine antibodies. The use of fluorescently labelled anti-phosphotyrosine antibodies or fluorescently labelled anti-phosphoserine or fluorescently labelled anti-phosphothreonine antibodies in the method of the present invention, allows real-time or semi real-time determination of the protein kinase activity and accordingly provides the possibility to express the protein kinase activity as the initial velocity of protein kinase derived from the activity over a certain period of incubation of the sample on the protein kinase substrates.

Accordingly, the present invention provides a phosphorylation profile obtained using detectably labelled antibodies.

The array of substrates is preferably a microarray of substrates wherein the substrates are immobilized onto a solid support or another carrier. The immobilization can be either the attachment or adherence of two or more substrate molecules to the surface of the carrier including attachment or adherence to the inner surface of said carrier in the case of e.g. a porous or flow-through solid support.

In a preferred embodiment of the present invention, the substrates are substrates for protein kinases.

More preferably the substrates used in the present invention are peptide substrates for protein kinases.

In a preferred embodiment of the present invention, the substrates are at least two substrates for protein kinases selected from the group consisting of the substrates for protein kinases with any of Seq.Id.No. 1 to 140, most particularly using at least two peptides selected from the group consisting of the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52. In a more preferred embodiment the substrates are the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52.

In a preferred embodiment of the present invention, the array of substrates is a flow-through array. The flow-through array as used herein could be made of any carrier material having oriented through-going channels as are generally known in the art, such as for example described in PCT patent publication WO 01/19517. Typically the carrier is made from a metal oxide, glass, silicon oxide or cellulose. In a particular embodiment the carrier material is made of a metal oxide selected from the group consisting of zinc oxide, zirconium oxide, tin oxide, aluminium oxide, titanium oxide and thallium; in a more particular embodiment the metal oxide consists of aluminium oxide.

In an alternative embodiment, the kinase activity in CSF can be determined in a monoplex and/or multiplex setup. This type of setup for measuring the kinase activity in CSF can for instance be based on the use of bead assays, or a multitude of single kinase assays or other methods known to those skilled in the art.

Alternatively, the method of the present invention can comprise steps where the kinase activity in CSF is measured by adding ATP and analyze the composition of the proteins by using methods known in the art such as, but not limited to 2D gel electrophoresis.

In a preferred embodiment of the present invention the phosphorylation profile is compared to a set of sample profiles of known neurological and psychiatric pathologies. These kinase activity profiles of known neurological and psychiatric pathologies can be present in the form of a database and the profiles can for instance be obtained from earlier conducted tests. The comparison can be used to ascertain the particular pathology of the cerebrospinal fluid being analysed. By comparing the activity profile with a large set of activity profiles from a database, this will render the method of the present invention more specific and precise.

Accordingly, within one embodiment of the present invention, a method is provided for analyzing CSF and determining the presence or development of a pathology, which can be neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease. The method comprises the steps of:
a) providing a sample of cerebrospinal fluid;
b) incubating said sample with ATP on an array of substrates;
c) obtaining a detectable phosphorylation profile, said profile resulting from the interaction of the cerebrospinal fluid sample with the array of substrates; and,
d) comparing the phosphorylation profile obtained in step (c) to a set of sample profiles of known neurological and psychiatric pathologies to ascertain the particular pathology of the cerebrospinal fluid being analysed.

In a preferred embodiment of the present invention, the pathology is preferably a neurological or a psychiatric disorder, preferably chosen from the group comprising neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease.

In an alternative embodiment of the present invention the method further comprises the presence of one or more protein kinase inhibitors in step b). In another embodiment the method further comprises the presence of one or more protein phosphatases in step b).

By providing a protein kinase inhibitor in the step where the kinase activity of the CSF sample is determined, it was surprisingly shown that the presence of the protein kinase inhibitor resulted in a more straightforward, less complicated and more discriminative activity profile. This surprising effect is due to the promiscuous characteristics of protein kinases. This results in a more efficient and less complicated analysis of the kinase activity profiles.

In another embodiment, the present invention relates to a method according to the present invention, use of an array according to the present invention, the use of a method according to the invention and/or use of a phosphorylation profile obtained by the method of the present invention, for the classification, diagnosis, prognosis and/or monitoring of neurological and psychiatric disorders. For example the method of the present invention can be used to diagnose neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease. The present disclosure also relates to the prediction and monitoring of treatment effects of said neurological and psychiatric disorders.

The present disclosure further relates to a method according to the present invention, an array according to the present invention and/or the use of a method according to the invention, for diagnostical, prognostical, and/or treatment predictive purposes. The kinase activity profiles obtained through the method of the present invention can for instance be used to assess the likelihood of a particular favourable or unfavourable outcome, susceptibility (or lack thereof) to a particular therapeutic regimen, etc. Therefore the method of the present invention also relates to the use of the method of the present invention to assess the susceptibility of a biological species having a specific disease state or cellular condition to a drug.

Therefore, the method of the present disclosure also relates to a method where CSF is used for drug discovery and/or drug screening comprising the steps of:
a) obtaining a sample of cerebrospinal fluid;
b) determining kinase activity of said sample by incubating said sample with ATP on an array of two or more substrates, preferably peptide substrates for protein kinases, thereby generating a kinase activity profile;
c) determining kinase activity of said sample by incubating said sample with ATP in the presence of a pharmacological compound and/or drug on an array of two or more substrates, preferably peptide substrates for protein kinases , thereby generating a kinase activity profile; and,
d) inferring the influence of said pharmacological compound and/or drug on the kinase activity profile, whereby an inhibition profile is generated by comparing the kinase activity profiles obtained in steps b) and c).

The present disclosure also relates to a method according to the present invention, an array according to the present invention, the use of the method of the present invention and/or a phosphorylation profile obtained by the method of the present invention for drug discovery and/or screening. By providing a method comprising the steps of:
a) obtaining a sample of cerebrospinal fluid;
b) determining kinase activity of said sample by incubating said sample with ATP on an array of two or more substrates, preferably peptide substrates for protein kinases , thereby generating a kinase activity profile;
c) determining kinase activity of said sample by incubating said sample with ATP in the presence of a pharmacological compound and/or drug on an array of two or more substrates, preferably peptide substrates for protein kinases , thereby generating a kinase activity profile;
d) inferring the influence of said pharmacological compound and/or drug on the kinase activity profile, whereby an inhibition profile is generated by comparing the kinase activity profiles obtained in step b) and c).

The pharmacological compound or drug as used in the present application can be any type of pharmacological active compound and/or drug and preferably a pharmacological compound and/or drug effecting kinase activity. The pharmacological compound and/or drug is preferably a kinase inhibitor, a kinase activator, a phosphatase inhibitor and/or a protease inhibitor.

This method enables the assessment of the pharmaceutical value and/or the clinical value of said pharmacological compound and/or drug and enables the assessment of the susceptibility to said pharmacological compound and/or drug of a biological species having a specific disease state or cellular condition. This method enables the prediction of the response of cells, tissues, organs and/or warm-blooded animals to said pharmacological compound and/or drug and determine the clinical outcome of a therapy with said pharmacological compound and/or drug. This method was found particular useful in the prediction of response to said pharmacological compound and/or drug, i.e. to enable the distinction between responders and non-responders in the treatment of cells, tissues, organs or warm-blooded animals with the pharmacological compound and/or drug to be tested, and in compound differentiation.

The pharmacological compound as used herein can be any kind of chemical substance for instance used in the treatment, cure, prevention, or diagnosis of disease or used to otherwise enhance physical or mental well-being. Preferably this drug is a kinase inhibitor.

The method of the present disclosure therefore relates to the use of the method of the present invention, wherein the inhibition profile is generated using an array of substrates comprising at least two peptides selected from the peptide substrates for protein kinases with sequence numbers 1 to 140.

The present disclosure relates in another embodiment to an array of substrates comprising at least two substrates for protein kinases selected from the group consisting of the substrates for protein kinases with any of Seq.Id.No. 1 to 140, most particularly using at least two peptides selected from the group consisting of the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52. In a more preferred embodiment the substrates are the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52.

In another embodiment of the present disclosure, a diagnostic kit, that enables performing the method of the present invention, is provided. Such a diagnostic kit would comprise at least one array containing at least two substrates for protein kinases selected from the group consisting of the substrates for protein kinases with any of Seq.Id.No. 1 to 140, most particularly using at least two peptides selected from the group consisting of the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52, and more preferably array contains substrates with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52. Said diagnostic kit would furthermore comprise ATP, BSA, buffer solutions and ingredients for detection of generated phosphorylation profiles.

The present disclosure also relates to a method for performing diagnosis on CSF samples, the method comprising: providing a computer platform comprising reference kinase activity profiles from CSF samples associated with neurological and/or psychiatric disorders and comparing the kinase activity profile of the CSF samples analysed using the method of the present invention with said reference profiles. The computer program can be provided on a data carrier comprising reference kinase activity profiles. Said computer program would enable performing diagnosis on the CSF samples. Furthermore, said computer program can be used for diagnostical purposes, prognostical purposes, for the prediction of the clinical outcome of a therapy and for treatment predictive purposes.

Furthermore, the present disclosure relates to a phosphorylation profile obtained by the method of the present invention, wherein said phosphorylation profile is specific for a certain neurological and/or psychiatric pathology. Furthermore, said phosphorylation profile can be used for diagnostical and/or prognostical purposes, the classification, and/or monitoring of disease progression of neurological and psychiatric disorders as well as the prediction and monitoring of treatment effects of said neurological and psychiatric disorders and/or for the prediction of the clinical outcome of a therapy.

### Examples

### Example 1:

The method of the present invention has been optimized to allow the measurement of the kinase activity in cerebrospinal fluid. CSF was obtained through a lumbar punction from patients suffering from Alzheimer's disease and control patients. A 10 µl aliquot of cerebrospinal fluid was added to a kinase incubation mixture containing ATP, and placed on a PamChip® STK array that was blocked with 2% BSA. After loading of the reaction mixtures onto Pamchip arrays comprising 140 substrates for protein kinases, incubation was commenced thereby measuring the kinase activity of the sample. As controls, arrays were incubated without ATP or without CSF. After 60 cycles of pumping the incubation mixture through the array, the mixture was removed and the array washed three times with PBS. Detection antibodies were added after removal of the wash buffer from the array. Images of the array were taken during the incubation of the array with the detection antibodies and after 30 cycles of incubation After 30 cycles of incubation and imaging, the antibody mixture was removed and the array was washed with PBS. Images were collected at different exposure times. Signals for each spot on the image were quantified. The signals intensities are used for further analysis.

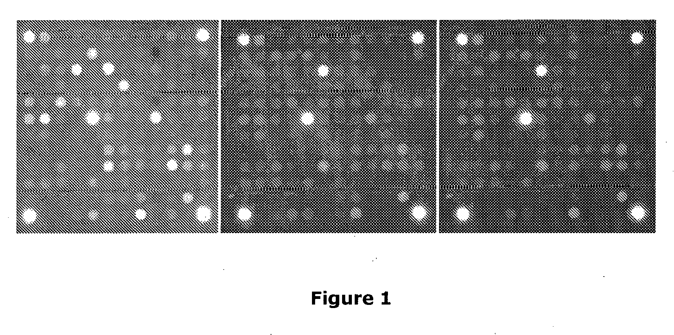

**Figure 1** shows the results of the incubation of the samples on a PamChip®. Whereas the samples containing no ATP (Fig. 1b) and no CSF (Fig. 1c) show identical patterns, the incubation with the sample containing CSF and ATP shows a unique phosphorylation profile. The corner spots are phosphorylated peptides, used as positive controls.

### Example 2:

CSF samples obtained from 3 classes of patients: control (2 samples), non-demented patients (7 samples) and demented patients (7 samples) were incubated as described in example 1 on PamChip® STK arrays. The fluorescence from the spots in the images was quantified for quantitative analysis.

For each peptide a one way ANOVA was performed to identify peptides with a significant difference between any of the 3 patient classes. Table 2 lists 44 out of the 140 peptides that have a probability p for equal means over the 3 patient classes of p < 0.01.

**Table 2: Peptides with p < 0.01 from the ANOVA analysis.**

| Seq.Id.No | Peptide |
|---|---|
| 80 | 'MK10_216_228_T218/T223/T228/S219/Y225' |
| 29 | 'CHK2_377_389_T378/T383/T387/T389/S379' |
| 9 | 'ADRB2_338_350_S345/S346/Y350' |
| 107 | 'PTN12_32_44_T40/T44/S39/Y42' |
| 108 | 'Q5HY18_106_111_S110' |
| 79 | 'MBP_225_238_T229/T232/S236' |
| 55 | 'HS90B_218_230_S225' |
| 24 | 'CFS1R_701_713_S713/Y708' |
| 19 | 'CBL_693_705_Y700' |
| 31 | 'CREB1_126_138_S129/S133/Y134' |
| 120 | 'RBL2_687_699_T694/S688/S690' |
| 36 | 'ELK1_410_422_T417/S411/S416/S422' |
| 6 | 'ACM5_498_510_T501/T505' |
| 57 | 'IPP1_28_40_T35/T38' |
| 95 | 'NR4A1_344_356_S351' |
| 99 | 'PDE5A_95_107_T96/T98/S102/S104' |
| 116 | 'RBL2_410_422_T417/T421/S413/S420' |
| 34 | 'ELK1_329_341_T336/S339/S341' |
| 12 | 'ANXA2_16_28_T18/S17/S21/S25/Y23' |
| 22 | 'CENPA_1_14_S7' |
| 129 | 'STK6_283_295_S283/S284/T287/T288/T292/Y295' |
| 32 | 'CSK21_355_367_T360/S356/S357/S362' |
| 18 | 'CALD1_746_758_T751/T753' |
| 106 | 'PTK6_436_448_T445/S442/S443/S446/Y447' |
| 47 | GRB2_427_439_T439/S427/S434' |
| 132 | 'TLE_242_254_T248/T249/S245' |
| 71 | 'KPCB_18_30_S24_A24S' |
| 38 | 'ERBB2_679_691_T686/Y685' |
| 125 | 'RYR1_4317_4329_T4324' |
| 127 | 'SRC_412_424_T419/Y418' |
| 117 | 'RBL2_632_644_T642/S639' |
| 62 | 'KCC1A_170_182_T177/T181/S173/S176' |
| 15 | 'CAC1C_1974_1986_S1975/S1981' |
| 51 | 'GYS2_1_14_T10/S6/S8/S11' |
| 128 | 'SRC8_CHICK_423_435_Y430' |
| 103 | 'PLM_76_88_T79/S82/S83/S88' |
| 8 | 'ADDB_706_718_T711/S713/S718' |
| 139 | 'VTNC_390_402_T400/S393/S397' |
| 83 | 'MPH6_140_152_T147' |
| 5 | 'ACM5_494_506_T501/T505/Y495' |
| 94 | 'NMDZ1_890_902_S890/S896/S897/T900/S901/T902' |
| 45 | 'GPR6_349_361_S350/S356/S358/S360' |
| 87 | 'MYPC3_268_280_T274/S269/S275' |
| 52 | 'H2B1B_26_39_S32/S36/S38' |

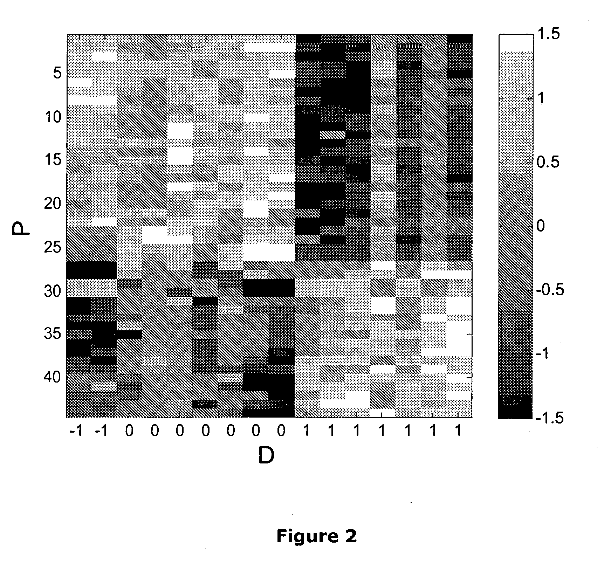

**Figure 2** is a map representing the phosphorylation patterns obtained with the CSF samples. The data only show the peptides selected by the One Way ANOVA analysis (table 2). The data was normalized per peptide by taking z-scores. On the horizontal axis (marked D) the 3 patient classes are indicated by -1 (control patients), 0 (non-demented patients) and 1 (demented patients). Along the vertical axis (marked P) the included peptides are sorted according to their z-score in the demented patient class. The results in Table 2 and Figure 2 show that a clear differentiation can be made between the CSF samples from the different patient classes.

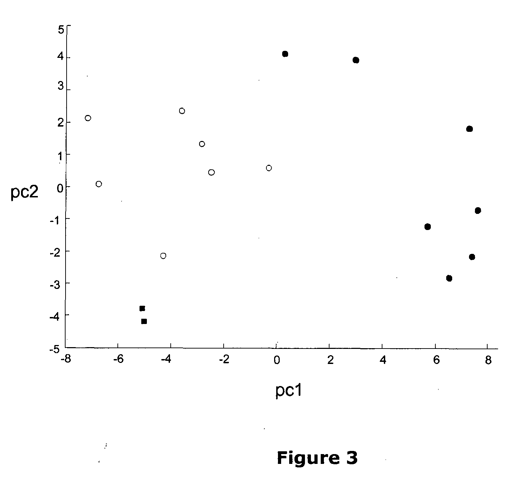

**Figure 3** shows the principal component analysis of the phosphorylation profiles in Figure 2. In Figure 3 the scores of each sample on the first two principal components is shown. The horizontal axis (marked pc1) represents the first principal component, the vertical axis (marked pc2) represents the second principal component. CSF samples obtained from control, non-demented and demented patients are represented by black squares, open circles, and black circles, respectively.

### Example 3:

The present example shows how the method of the present invention can be used for the identification of protein kinases involved in Alzheimer's disease.

A 10 µl aliquot of freshly frozen post mortem cerebrospinal fluid was added to a kinase incubation mixture containing ATP and detection antibodies and placed on a PamChip® STK array that was blocked with 2% BSA. After loading of the reaction mixtures onto Pamchip arrays comprising 140 substrates for protein kinases, incubation was commenced thereby measuring the kinase activity of the sample. As controls, arrays were incubated without ATP or without CSF. After 60 cycles of pumping the incubation mixture through the array, the mixture was removed and the array washed three times with PBS. A mixture of detection antibodies was added to the array and pumped up and down. Images were made at intervals of ten cycles of pumping. After 60 cycles of incubation and imaging, the detection mixture was removed and the array was washed with PBS. Images were collected at different exposure times. Signals for each spot on the image were quantified. The signals intensities were used for further analysis. The signals obtained in the incubations without ATP were subtracted from the signals obtained in the presence of ATP. Table 2 illustrates the differences in phosphorylation of peptide markers with Seq. Id. Nos. 4, 5, 6, 43, 63, 77, 97 and 111 in samples from non-demented (CSF 1-3) and demented patients (CSF 4-6).

**Table 3**

| | **CSF1** | **CSF2** | **CSF3** | **CSF4** | **CSF5** | **CSF6** |
|---|---|---|---|---|---|---|
| | Non-demented control, br 0,O | | | Alzheimer's disease, br. 6,C | | |
| Seq. Id. No. 4 | -134 | -77 | 62 | 663 | 366 | 548 |
| Seq. Id. No. 5 | -123 | 457 | 546 | 1415 | 1293 | 1361 |
| Seq. Id. No. 6 | 56 | 228 | 85 | 1291 | 920 | 664 |
| Seq. Id. No. 43 | 19 | 9 | 7 | 552 | 533 | 503 |
| Seq. Id. No. 63 | -882 | -124 | -398 | 466 | 327 | 898 |
| Seq. Id. No. 77 | -220 | -67 | 78 | 661 | 580 | 404 |
| Seq. Id. No. 97 | 116 | 222 | 134 | 268 | 417 | 515 |
| Seq. Id. No. 111 | 882 | 2856 | 633 | 4771 | 4429 | 3756 |

The measurements of the kinase activity of post mortem CSF samples obtained from Alzheimer patients and non-demented cases indicated that the phosphorylation profiles on a PamChip STK array were clearly different. More specifically, the difference in kinase activity was found to be associated with protein kinases of the IRAK family. This conclusion was confirmed by comparing the phosphorylation profiles of these samples and with phosphorylation profiles generated by recombinant IRAK-4 kinase. The presence of IRAK-4 in post mortem brain tissue has further been confirmed by Western blot analyses using commercially available antibodies.

The involvement of protein kinases of the IRAK family in Alzheimer's disease was further confirmed by determining the IRAK-4 expression by immunohistochemistry using post mortem brain tissue derived from 22 different cases, 11 cases of Alzheimer's disease and 11 non-demented control cases. Immunohistochemical staining shows localization of IRAK-4 in astrocytes and microglia. These results were obtained using different commercially available antibodies.

The IRAK-4 expression in astrocytes was further validated in vitro. Western blot analysis and immunocytochemistry shows presence of IRAK-4 in U373 cells (astrocytoma cell line) and primary human adult astrocytes obtained after surgery or isolated from post mortem derived brain tissue. Staining for the presence of IRAK-4 on Western blot and immunocytochemistry was performed using commercially available antibodies.

### Example 4:

The present example shows how the protein kinases involved in Alzheimer's disease identified in Example 3 provide targets for the treatment of Alzheimer's Disease.

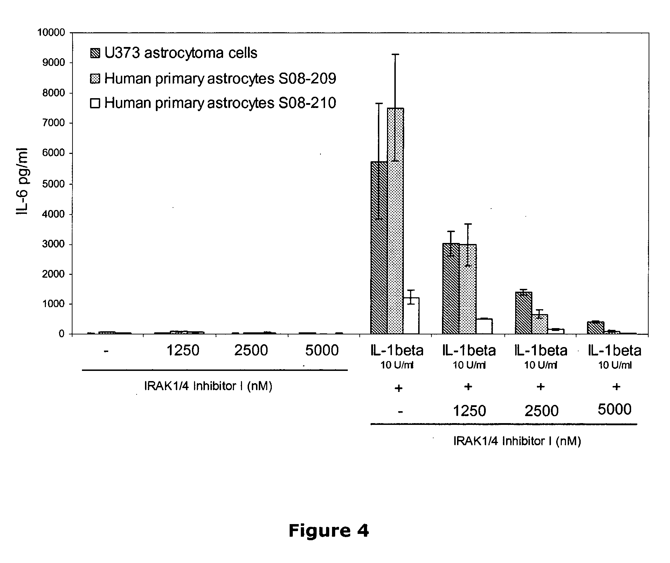

IL-1β has been implicated in both the initiation and propagation of neuroinflammatory changes seen in Alzheimer's disease patients. One of the main mechanisms of IL-1β is the induction of interleukin 6 (IL-6) secretion, which propagates the neuroinflammatory response in Alzheimer's disease patients. Using primary human adult astrocytes this inflammatory response as well as modulators (i.e. potential drugs for therapy) can be studied in vitro. Figure 4 shows the secretion of IL-6 by U373 astrocytoma cells and human primary astrocytes after 6 hours in culture. In the presence of IL-1β (10 U/ml) these cells secrete IL-6 in the culture supernatant, which can be measured by ELISA. The presence of IRAK1/4 inhibitor I dose-dependently inhibits IL-6 secretion. Cells were incubated with or without IL-1β (10 U/ml) for 6 hours in the presence of different concentration of IRAK1/4 inhibitor I (1250, 2500, or 5000 nM). IL-6 levels (pg/ml) in the culture supernatants were determined by ELISA. Shown are mean levels +/- S.D. of three replicate stimulations.

This indicates that inhibition of IRAK1/4 could reduce the neuroinflammatory response in Alzheimer patients.

## Claims

1. Ex vivo method for measuring protein kinase activity in a cerebrospinal fluid, said method comprising the steps of:
a) providing a sample of cerebrospinal fluid;
b) incubating said sample with ATP on an array of substrates, wherein said substrates are peptide substrates for protein kinases; and,
c) obtaining a detectable phosphorylation profile, said profile resulting from the interaction of the cerebrospinal fluid sample with the array of substrates.

2. Method according to claim 1, wherein said substrates are at least two peptide substrates for protein kinases chosen from the group consisting of the substrates for protein kinases with Seq.Id.No. 1 to 140.

3. Method according to claim 1, wherein said substrates are at least two peptide substrates for protein kinases chosen from the group consisting of the peptides with any of Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52.

4. Method according to any of claims 1 to 3, wherein said array is a flow-through array.

5. Method according to any of claims 1 to 4, further comprising a step wherein the phosphorylation profile obtained in step (c) is compared to a set of sample profiles of known neurological and psychiatric pathologies to ascertain the particular pathology of the cerebrospinal fluid being analysed.

6. Method according to claim 5, wherein the pathology is selected from the group comprising neurological and/or psychiatric disorders, such as Alzheimer's disease, Huntington's disease, Parkinson's disease, Creutzfeldt-Jakob disease and other prion diseases, fronto temporal dementia, dystonia, ataxia's, schizophrenia, epilepsy, depression, brain tumors, brain irradiation, head trauma, multiple sclerosis, white matter disorders, metabolic disorders, acute and chronic encephalitic and vascular disease.

7. Method according to any of claims 1 to 6, wherein said phosphorylation profile is specific for a certain neurological and/or psychiatric pathology.

8. Method according to any of claims 1 to 7, for the classification, diagnosis, prognosis and/or monitoring of neurological and psychiatric disorders as well as the prediction and monitoring of treatment effects of said neurological and psychiatric disorders.

9. Method according to any of claims 1 to 8, for drug discovery and/or screening.

## Patentansprüche

1. Ex-vivo-Verfahren zum Messen der Proteinkinaseaktivität in Liquor cerebrospinalis, wobei das Verfahren die folgenden Schritte umfasst:
a) Bereitstellen einer Liquor cerebrospinalis Probe;
b) Inkubieren der Probe mit ATP auf einer Anordnung von Substraten, wobei es sich bei den Substraten um Peptidsubstrate für Proteinkinasen handelt; und,
c) Erhalten eines nachweisbaren Phosphorylierungsprofils, wobei das Profil aus der Wechselwirkung der Liquor cerebrospinalis probe mit der Anordnung von Substraten resultiert.

2. Verfahren nach Anspruch 1, wobei es sich bei den Substraten um mindestens zwei Peptidsubstrate für Proteinkinasen handelt, ausgewählt aus der Gruppe bestehend aus den Substraten für Proteinkinasen mit Seq.Id.No. 1 bis 140.

3. Verfahren nach Anspruch 1, wobei es sich bei den Substraten um mindestens zwei Peptidsubstrate für Proteinkinasen handelt, ausgewählt aus der Gruppe bestehend aus den Peptiden mit einer der folgenden Seq.Id.No. 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 und 52.

4. Verfahren nach einem der Ansprüche 1 bis 3, wobei es sich bei der Anordnung um eine Durchflussanordnung handelt.

5. Verfahren nach einem der Ansprüche 1 bis 4, ferner umfassend einen Schritt, wobei das in Schritt c) erhaltene Phosphorylierungsprofil mit einem Satz von Probenprofilen bekannter neurologischer und psychiatrischer Pathologien verglichen wird, die spezielle Pathologie des analysierten Liquors cerebrospinalis zu ermitteln.

6. Verfahren nach Anspruch 5, wobei die Pathologie ausgewählt ist aus der Gruppe umfassend neurologische und/oder psychiatrische Störungen, wie etwa Alzheimer-Krankheit, Huntington-Krankheit, Parkinson-Krankheit, Creutzfeldt-Jakob-Krankheit und andere Prionenkrankheiten, Frontotemporale Demenz, Dystonie, Ataxien, Schizophrenie, Epilepsie, Depression, Hirntumore, Hirnbestrahlung, Kopftrauma, multiple Sklerose, Störungen der weißen Substanz, Stoffwechselstörungen, akute und chronische enzephalitische und vaskuläre Krankheit.

7. Verfahren nach einem der Ansprüche 1 bis 6, wobei das Phosphorylierungsprofil für eine bestimmte neurologische und/oder psychiatrische Pathologie spezifisch ist.

8. Verfahren nach einem der Ansprüche 1 bis 7 zur Klassifizierung, Diagnose, Prognose und/oder Überwachung von neurologischen und psychiatrischen Störungen sowie zur Vorhersage und Überwachung von Behandlungseffekten dieser neurologischen und psychiatrischen Störungen.

9. Verfahren nach einem der Ansprüche 1 bis 8 zum Entdecken und/oder Screenen von Arzneimitteln.

## Revendications

1. Procédé ex-vivo pour mesurer l'activité d'une protéine kinase dans un fluide céphalo-rachidien, ledit procédé comprenant les étapes de:
a) fourniture d'un échantillon de fluide céphalo-rachidien;
b) incubation dudit échantillon avec de l'ATP sur une gamme de substrats, dans laquelle lesdits substrats sont des substrats peptidiques pour les protéines kinase; et,
c) obtention d'un profil de phosphorylation détectable, ledit profil résultant de l'interaction de l'échantillon de fluide céphalo-rachidien avec la gamme de substrats.

2. Procédé selon la revendication 1, dans lequel lesdits substrats sont au moins deux substrats peptidiques pour les protéines kinases choisies dans le groupe constitué des substrats pour les protéines kinases avec une Séq.Id. N°1 à 140.

3. Procédé selon la revendication 1, dans lequel lesdits substrats sont au moins deux substrats peptidiques pour les protéines kinases choisies dans le groupe constitué de peptides avec une séquence Séq.Id. N° 80, 29, 9, 107, 108, 79, 55, 24, 19, 31, 120, 36, 6, 57, 95, 99, 116, 34, 12, 22, 129, 32, 18, 106, 47, 132, 71, 38, 125, 127, 117, 62, 15, 51, 128, 103, 8, 139, 83, 5, 94, 45, 87 and 52.

4. Procédé selon l'une quelconque des revendications 1 à 3, dans lequel ladite gamme est une gamme intermédiaire.

5. Procédé selon l'une quelconque des revendications 1 à 4, comprenant en outre une étape dans laquelle le profil de phosphorylation obtenu à l'étape (c) est comparé à un ensemble de profils d'échantillonnage des pathologies neurologiques et psychiatriques connues pour déterminer la pathologie particulière du liquide céphalo-rachidien en cours d'analyse.

6. Procédé selon la revendication 5, dans lequel la pathologie est choisie dans le groupe comprenant les troubles neurologiques et/ou psychiatriques, tels que la maladie d'Alzheimer, la maladie de Huntington, la maladie de Parkinson, la maladie de Creutzfeldt-Jakob et autres maladies à prions, la démence fronto-temporelle, la dystonie, l'ataxie, la schizophrénie, l'épilepsie, la dépression, les tumeurs cérébrales, l'irradiation du cerveau, les traumatismes crâniens, la sclérose en plaques, les troubles de la substance blanche, les troubles métaboliques, l'encéphalite aiguë et chronique ainsi que les maladies vasculaires.

7. Procédé selon l'une quelconque des revendications 1 à 6, dans lequel ledit profil de phosphorylation est spécifique à une certaine pathologie neurologique et/ou psychiatrique.

8. Procédé selon l'une quelconque des revendications 1 à 7, pour la classification, le diagnostic, le pronostic et/ou le suivi de troubles neurologiques et/ou psychiatriques, ainsi que la prévision et le suivi d'effets du traitement desdits troubles neurologiques et psychiatriques.

9. Procédé selon l'une quelconque des revendications 1 à 8, pour la découverte de médicaments et/ou le dépistage.


### SEQUENCE LISTING

<110> PamGene B.V.
   Vereniging voor christelijk hoger onderwijs, wetenschappelijk onderzoek en patientenzorg
<120> Measurement of protein kinase activity in cerebrospinal fluid for diagnosis of neurological and psychiatric disorders
<130> PAM-048-PCT
<160> 140
<170> PatentIn version 3.3
<210> 1
   <211> 12
   <212> PRT
   <213> Artificial
<220>
   <223> peptide kinase substrate
<400> 1
   Thr Glu Trp Arg Ser Val Thr Leu Pro Arg Asp Leu
   1               5                   10
<210> 2
   <211> 13
   <212> PRT
   <213> Artificial
<220>
   <223> peptide kinase substrate
<400> 2
   Cys Asn Lys Ala Phe Arg Asp Thr Phe Arg Leu Leu Leu
   1               5                   10
<210> 3
   <211> 13
   <212> PRT
   <213> Artificial
<220>
   <223> peptide kinase substrate
<400> 3
   Lys Ile Pro Lys Arg Pro Gly Ser Val His Arg Thr Pro
   1 5 10
<210> 4
   <211> 13
   <212> PRT
   <213> Artificial
<220>
   <223> peptide kinase substrate
<400> 4
   Cys Asn Ala Thr Phe Lys Lys Thr Phe Arg His Leu Leu
   1 5 10
<210> 5
   <211> 13
   <212> PRT
   <213> Artificial
<220>
   <223> peptide kinase substrate
<400> 5
   Cys Tyr Ala Leu Cys Asn Arg Thr Phe Arg Lys Thr Phe
   1 5 10
<210> 6
   <211> 13
   <212> PRT
   <213> Artificial
<220>
   <223> peptide kinase substrate
<400> 6
   Cys Asn Arg Thr Phe Arg Lys Thr Phe Lys Met Leu Leu
   1 5 10
<210> 7
   <211> 13
   <212> PRT
   <213> Artificial
<220>
   <223> peptide kinase substrate
<400> 7
   Gly Ser Pro Ser Lys Ser Pro Ser Lys Lys Lys Lys Lys
   1 5 10
<210> 8
   <211> 13
   <212> PRT
   <213> Artificial
<220>
   <223> peptide kinase substrate
<400> 8
   Lys Lys Lys Phe Arg Thr Pro Ser Phe Leu Lys Lys Ser
   1 5 10
<210> 9
   <211> 13
   <212> PRT
   <213> Artificial
<220>
   <223> peptide kinase substrate
<400> 9
   Glu Leu Leu Cys Leu Arg Arg Ser Ser Leu Lys Ala Tyr
   1 5 10
<210> 10
   <211> 13
   <212> PRT
   <213> Artificial
<220>
   <223> peptide kinase substrate
<400> 10
   Lys Asp Gly Ala Thr Met Lys Thr Phe Cys Gly Thr Pro
   1 5 10
<210> 11
   <211> 13
   <212> PRT
   <213> Artificial
<220>
   <223> peptide kinase substrate
<400> 11
   Ala Gly Glu Arg Arg Lys Gly Thr Asp Val Asn Val Phe
   1 5 10
<210> 12
   <211> 13
   <212> PRT
   <213> Artificial
<220>
   <223> peptide kinase substrate
<400> 12
   His Ser Thr Pro Pro Ser Ala Tyr Gly Ser Val Lys Ala
   1               5                   10
<210> 13
   <211> 13
   <212> PRT
   <213> Artificial
<220>
   <223> peptide kinase substrate
<400> 13
   Val Ala Asp Gln Thr Pro Thr Pro Thr Arg Phe Leu Lys
   1               5                   10
<210> 14
   <211> 13
   <212> PRT
   <213> Artificial
<220>
   <223> peptide kinase substrate
<400> 14
   Glu Arg Ser Lys Thr Val Thr Ser Phe Tyr Asn Gln Ser
   1               5                   10
<210> 15
   <211> 13
   <212> PRT
   <213> Artificial
<220>
   <223> peptide kinase substrate
<400> 15
   Ala Ser Leu Gly Arg Arg Ala Ser Phe His Leu Glu Cys
   1               5                   10
<210> 16
   <211> 13
   <212> PRT
   <213> Artificial
<220>
   <223> peptide kinase substrate
<400> 16
   Leu Glu Asp Ile Lys Arg Leu Thr Pro Arg Phe Thr Leu
   1               5                   10
<210> 17
   <211> 13
   <212> PRT
   <213> Artificial
<220>
   <223> peptide kinase substrate
<400> 17
   Lys Thr Pro Asp Gly Asn Lys Ser Pro Ala Pro Lys Pro
   1               5                   10
<210> 18
   <211> 13
   <212> PRT
   <213> Artificial
<220>
   <223> peptide kinase substrate
<400> 18
   Ile Asn Glu Trp Leu Thr Lys Thr Pro Asp Gly Asn Lys
   1               5                   10
<210> 19
   <211> 13
   <212> PRT
   <213> Artificial
<220>
   <223> peptide kinase substrate
<400> 19
   Glu Gly Glu Glu Asp Thr Glu Tyr Met Thr Pro Ser Ser
   1               5                   10
<210> 20
   <211> 16
   <212> PRT
   <213> Artificial
<220>
   <223> peptide kinase substrate
<400> 20
   Gly Ile Pro Ile Arg Val Tyr Thr His Glu Val Val Thr Leu Trp Tyr
   1               5                   10                  15
<210> 21
   <211> 13
   <212> PRT
   <213> Artificial
<220>
   <223> peptide kinase substrate
<400> 21
   Gly Ser Pro Asn Arg Ala Tyr Thr His Gln Val Val Thr
   1               5                   10
<210> 22
   <211> 14
   <212> PRT
   <213> Artificial
<220>
   <223> peptide kinase substrate
<400> 22
   Met Gly Pro Arg Arg Arg Ser Arg Lys Pro Glu Ala Pro Arg
   1               5                   10
<210> 23
   <211> 13
   <212> PRT
   <213> Artificial
<220>
   <223> peptide kinase substrate
<400> 23
   His His Lys Leu Val Leu Pro Ser Asn Thr Pro Asn Val
   1               5                   10
<210> 24
   <211> 13
   <212> PRT
   <213> Artificial
<220>
   <223> peptide kinase substrate
<400> 24
   Asn Ile His Leu Glu Lys Lys Tyr Val Arg Arg Asp Ser
   1               5                   10
<210> 25
   <211> 13
   <212> PRT
   <213> Artificial
<220>
   <223> peptide kinase substrate
<400> 25
   Glu Pro Leu Glu Arg Arg Leu Ser Leu Val Pro Asp Ser
   1 5 10
<210> 26
   <211> 13
   <212> PRT
   <213> Artificial
<220>
   <223> peptide kinase substrate
<400> 26
   Leu Gln Ala Arg Arg Arg Gln Ser Val Leu Asn Leu Met
   1 5 10
<210> 27
   <211> 13
   <212> PRT
   <213> Artificial
<220>
   <223> peptide kinase substrate
<400> 27
   Ile His Arg Lys Thr Thr Ala Ser Thr Arg Lys Val Ser
   1 5 10
<210> 28
   <211> 13
   <212> PRT
   <213> Artificial
<220>
   <223> peptide kinase substrate
<400> 28
   Gln Cys Ala Leu Cys Arg Arg Ser Thr Thr Asp Cys Gly
   1 5 10
<210> 29
   <211> 13
   <212> PRT
   <213> Artificial
<220>
   <223> peptide kinase substrate
<400> 29
   Glu Thr Ser Leu Met Arg Thr Leu Cys Gly Thr Pro Thr
   1 5 10
<210> 30
   <211> 13
   <212> PRT
   <213> Artificial
<220>
   <223> peptide kinase substrate
<400> 30
   Asp Met Lys Val Arg Lys Ser Ser Thr Pro Glu Glu Val
   1               5                   10
<210> 31
   <211> 13
   <212> PRT
   <213> Artificial
<220>
   <223> peptide kinase substrate
<400> 31
   Glu Ile Leu Ser Arg Arg Pro Ser Tyr Arg Lys Ile Leu
   1               5                   10
<210> 32
   <211> 13
   <212> PRT
   <213> Artificial
<220>
   <223> peptide kinase substrate
<400> 32
   Ile Ser Ser Val Pro Thr Pro Ser Pro Leu Gly Pro Leu
   1               5                   10
<210> 33
   <211> 13
   <212> PRT
   <213> Artificial
<220>
   <223> peptide kinase substrate
<400> 33
   His Phe Asp Glu Arg Asp Lys Thr Ser Arg Asn Met Arg
   1               5                   10
<210> 34
   <211> 13
   <212> PRT
   <213> Artificial
<220>
   <223> peptide kinase substrate
<400> 34
   Gly Gly Pro Gly Pro Glu Arg Thr Pro Gly Ser Gly Ser
   1 5 10
<210> 35
   <211> 13
   <212> PRT
   <213> Artificial
<220>
   <223> peptide kinase substrate
<400> 35
   Leu Leu Pro Thr His Thr Leu Thr Pro Val Leu Leu Thr
   1 5 10
<210> 36
   <211> 13
   <212> PRT
   <213> Artificial
<220>
   <223> peptide kinase substrate
<400> 36
   Ile Ser Val Asp Gly Leu Ser Thr Pro Val Val Leu Ser
   1 5 10
<210> 37
   <211> 13
   <212> PRT
   <213> Artificial
<220>
   <223> peptide kinase substrate
<400> 37
   Leu Leu Asn Lys Arg Arg Gly Ser Val Pro Ile Leu Arg
   1 5 10
<210> 38
   <211> 13
   <212> PRT
   <213> Artificial
<220>
   <223> peptide kinase substrate
<400> 38
   Gln Gln Lys Ile Arg Lys Tyr Thr Met Arg Arg Leu Leu
   1 5 10
<210> 39
   <211> 13
   <212> PRT
   <213> Artificial
<220>
   <223> peptide kinase substrate
<400> 39
   Gly Glu Ala Gly Gly Pro Leu Thr Pro Arg Arg Val Ser
   1               5                   10
<210> 40
   <211> 13
   <212> PRT
   <213> Artificial
<220>
   <223> peptide kinase substrate
<400> 40
   Gly Gly Arg Glu Arg Leu Ala Ser Thr Asn Asp Lys Gly
   1               5                   10
<210> 41
   <211> 13
   <212> PRT
   <213> Artificial
<220>
   <223> peptide kinase substrate
<400> 41
   Asn Pro Leu Met Arg Arg Asn Ser Val Thr Pro Leu Ala
   1               5                   10
<210> 42
   <211> 13
   <212> PRT
   <213> Artificial
<220>
   <223> peptide kinase substrate
<400> 42
   Leu Asn Val Ala Ala Val Asn Thr His Arg Asp Arg Pro
   1               5                   10
<210> 43
   <211> 13
   <212> PRT
   <213> Artificial
<220>
   <223> peptide kinase substrate
<400> 43
   Glu Phe Pro Ser Arg Gly Lys Ser Ser Ser Tyr Ser Lys
   1 5 10
<210> 44
   <211> 13
   <212> PRT
   <213> Artificial
<220>
   <223> peptide kinase substrate
<400> 44
   Gln Ser Arg Pro Arg Ser Cys Thr Trp Pro Leu Gln Arg
   1 5 10
<210> 45
   <211> 13
   <212> PRT
   <213> Artificial
<220>
   <223> peptide kinase substrate
<400> 45
   Gln Ser Lys Val Pro Phe Arg Ser Arg Ser Pro Ser Glu
   1 5 10
<210> 46
   <211> 13
   <212> PRT
   <213> Artificial
<220>
   <223> peptide kinase substrate
<400> 46
   Pro Lys Leu Gly Arg Arg His Ser Met Glu Asn Met Glu
   1 5 10
<210> 47
   <211> 13
   <212> PRT
   <213> Artificial
<220>
   <223> peptide kinase substrate
<400> 47
   Ser Arg Leu Arg Arg Arg Ala Ser Gln Leu Lys Ile Thr
   1 5 10
<210> 48
   <211> 13
   <212> PRT
   <213> Artificial
<220>
   <223> peptide kinase substrate
<400> 48
   Glu Lys Met Trp Ala Phe Met Ser Ser Arg Gln Gln Thr
   1               5                   10
<210> 49
   <211> 13
   <212> PRT
   <213> Artificial
<220>
   <223> peptide kinase substrate
<400> 49
   Phe Met Ser Ser Arg Arg Gln Ser Val Leu Val Lys Ser
   1               5                   10
<210> 50
   <211> 13
   <212> PRT
   <213> Artificial
<220>
   <223> peptide kinase substrate
<400> 50
   Lys Lys Pro Arg Arg Lys Asp Thr Pro Ala Leu His Ile
   1               5                   10
<210> 51
   <211> 13
   <212> PRT
   <213> Artificial
<220>
   <223> peptide kinase substrate
<400> 51
   Met Leu Arg Gly Arg Ser Leu Ser Val Thr Ser Leu Gly
   1               5                   10
<210> 52
   <211> 14
   <212> PRT
   <213> Artificial
<220>
   <223> peptide kinase substrate
<400> 52
   Gly Lys Lys Arg Lys Arg Ser Arg Lys Glu Ser Tyr Ser Ile
   1 5 10
<210> 53
   <211> 13
   <212> PRT
   <213> Artificial
<220>
   <223> peptide kinase substrate
<400> 53
   Asp Gly Lys Lys Arg Lys Arg Ser Arg Lys Glu Ser Tyr
   1 5 10
<210> 54
   <211> 16
   <212> PRT
   <213> Artificial
<220>
   <223> peptide kinase substrate
<400> 54
   Arg Thr Lys Gln Thr Ala Arg Lys Ser Thr Gly Gly Lys Ala Pro Arg
   1 5 10 15
<210> 55
   <211> 13
   <212> PRT
   <213> Artificial
<220>
   <223> peptide kinase substrate
<400> 55
   Lys Glu Arg Glu Lys Glu Ile Ser Asp Asp Glu Ala Glu
   1 5 10
<210> 56
   <211> 13
   <212> PRT
   <213> Artificial
<220>
   <223> peptide kinase substrate
<400> 56
   Lys Lys Asn Gly Arg Ile Leu Thr Leu Pro Arg Ser Asn
   1 5 10
<210> 57
   <211> 13
   <212> PRT
   <213> Artificial
<220>
   <223> peptide kinase substrate
<400> 57
   Gln Ile Arg Arg Arg Arg Pro Thr Pro Ala Thr Leu Val
   1               5                   10
<210> 58
   <211> 13
   <212> PRT
   <213> Artificial
<220>
   <223> peptide kinase substrate
<400> 58
   Leu Glu His Val Thr Arg Arg Thr Leu Ser Met Asp Lys
   1               5                   10
<210> 59
   <211> 13
   <212> PRT
   <213> Artificial
<220>
   <223> peptide kinase substrate
<400> 59
   Ser Arg Phe Asn Arg Arg Val Ser Val Cys Ala Glu Thr
   1               5                   10
<210> 60
   <211> 13
   <212> PRT
   <213> Artificial
<220>
   <223> peptide kinase substrate
<400> 60
   Asn Arg Phe Thr Arg Arg Ala Ser Val Cys Ala Glu Ala
   1               5                   10
<210> 61
   <211> 15
   <212> PRT
   <213> Artificial
<220>
   <223> peptide kinase substrate
<400> 61
   Val Lys Gly Arg Thr Trp Thr Leu Cys Gly Thr Pro Glu Tyr Leu
   1 5 10 15
<210> 62
   <211> 13
   <212> PRT
   <213> Artificial
<220>
   <223> peptide kinase substrate
<400> 62
   Asp Pro Gly Ser Val Leu Ser Thr Ala Cys Gly Thr Pro
   1 5 10
<210> 63
   <211> 12
   <212> PRT
   <213> Artificial
<220>
   <223> peptide kinase substrate
<400> 63
   Val Ala Ser Met Met His Arg Gln Glu Thr Val Glu
   1 5 10
<210> 64
   <211> 13
   <212> PRT
   <213> Artificial
<220>
   <223> peptide kinase substrate
<400> 64
   Asp Ser Asp Leu Ser Arg Arg Ser Ser Ser Thr Met Ser
   1 5 10
<210> 65
   <211> 13
   <212> PRT
   <213> Artificial
<220>
   <223> peptide kinase substrate
<400> 65
   Pro Asp Leu Lys Lys Ser Arg Ser Ala Ser Thr Ile Ser
   1 5 10
<210> 66
   <211> 13
   <212> PRT
   <213> Artificial
<220>
   <223> peptide kinase substrate
<400> 66
   Glu Glu Leu Arg Lys Ala Arg Ser Asn Ser Thr Leu Ser
   1               5                   10
<210> 67
   <211> 13
   <212> PRT
   <213> Artificial
<220>
   <223> peptide kinase substrate
<400> 67
   Ala Asn Arg Glu Arg Arg Pro Ser Tyr Leu Pro Thr Pro
   1               5                   10
<210> 68
   <211> 13
   <212> PRT
   <213> Artificial
<220>
   <223> peptide kinase substrate
<400> 68
   Lys Trp Thr Lys Arg Thr Leu Ser Glu Thr Ser Ser Ser
   1               5                   10
<210> 69
   <211> 13
   <212> PRT
   <213> Artificial
<220>
   <223> peptide kinase substrate
<400> 69
   Leu Asp Ile Pro Thr Gly Thr Thr Pro Gln Arg Lys Ser
   1               5                   10
<210> 70
   <211> 13
   <212> PRT
   <213> Artificial
<220>
   <223> peptide kinase substrate
<400> 70
   Gln Val Glu Phe Arg Arg Leu Ser Ile Ser Ala Glu Ser
   1 5 10
<210> 71
   <211> 13
   <212> PRT
   <213> Artificial
<220>
   <223> peptide kinase substrate
<400> 71
   Arg Phe Ala Arg Lys Gly Ser Leu Arg Gln Lys Asn Val
   1 5 10
<210> 72
   <211> 14
   <212> PRT
   <213> Artificial
<220>
   <223> peptide kinase substrate
<400> 72
   Ala Glu Asn Phe Asp Arg Phe Phe Thr Arg His Pro Pro Val
   1 5 10
<210> 73
   <211> 13
   <212> PRT
   <213> Artificial
<220>
   <223> peptide kinase substrate
<400> 73
   Gly Lys Lys Thr Lys Phe Ala Ser Asp Asp Glu His Asp
   1 5 10
<210> 74
   <211> 13
   <212> PRT
   <213> Artificial
<220>
   <223> peptide kinase substrate
<400> 74
   Gly Gly Pro Thr Thr Pro Leu Ser Pro Thr Arg Leu Ser
   1 5 10
<210> 75
   <211> 13
   <212> PRT
   <213> Artificial
<220>
   <223> peptide kinase substrate
<400> 75
   Asp Ala Glu Asn Arg Leu Gln Thr Met Lys Glu Glu Leu
   1 5 10
<210> 76
   <211> 13
   <212> PRT
   <213> Artificial
<220>
   <223> peptide kinase substrate
<400> 76
   Lys Lys Lys Lys Lys Arg Phe Ser Phe Lys Lys Ser Phe
   1 5 10
<210> 77
   <211> 13
   <212> PRT
   <213> Artificial
<220>
   <223> peptide kinase substrate
<400> 77
   Phe Lys Lys Ser Phe Lys Leu Ser Gly Phe Ser Phe Lys
   1 5 10
<210> 78
   <211> 13
   <212> PRT
   <213> Artificial
<220>
   <223> peptide kinase substrate
<400> 78
   His Phe Phe Lys Asn Ile Val Thr Pro Arg Thr Pro Pro
   1 5 10
<210> 79
   <211> 13
   <212> PRT
   <213> Artificial
<220>
   <223> peptide kinase substrate
<400> 79
   Lys Asn Ile Val Thr Pro Arg Thr Pro Pro Pro Ser Gln
   1 5 10
<210> 80
   <211> 13
   <212> PRT
   <213> Artificial
<220>
   <223> peptide kinase substrate
<400> 80
   Ala Gly Thr Ser Phe Met Met Thr Pro Tyr Val Val Thr
   1 5 10
<210> 81
   <211> 13
   <212> PRT
   <213> Artificial
<220>
   <223> peptide kinase substrate
<400> 81
   Gly Asp Ala Ala Glu Thr Pro Pro Arg Pro Arg Thr Pro
   1 5 10
<210> 82
   <211> 13
   <212> PRT
   <213> Artificial
<220>
   <223> peptide kinase substrate
<400> 82
   Pro Pro Arg Pro Arg Thr Pro Gly Arg Pro Leu Ser Ser
   1 5 10
<210> 83
   <211> 13
   <212> PRT
   <213> Artificial
<220>
   <223> peptide kinase substrate
<400> 83
   Glu Asp Glu Asn Gly Asp Ile Thr Pro Ile Lys Ala Lys
   1 5 10
<210> 84
   <211> 13
   <212> PRT
   <213> Artificial
<220>
   <223> peptide kinase substrate
<400> 84
   Arg Ser Gly Leu Tyr Arg Ser Pro Ser Met Pro Glu Asn
   1               5                   10
<210> 85
   <211> 13
   <212> PRT
   <213> Artificial
<220>
   <223> peptide kinase substrate
<400> 85
   Asp Asn Thr Pro His Thr Pro Thr Pro Phe Lys Asn Ala
   1               5                   10
<210> 86
   <211> 13
   <212> PRT
   <213> Artificial
<220>
   <223> peptide kinase substrate
<400> 86
   Lys Lys Phe Glu Leu Leu Pro Thr Pro Pro Leu Ser Pro
   1               5                   10
<210> 87
   <211> 13
   <212> PRT
   <213> Artificial
<220>
   <223> peptide kinase substrate
<400> 87
   Leu Ser Ala Phe Arg Arg Thr Ser Leu Ala Gly Gly Gly
   1               5                   10
<210> 88
   <211> 13
   <212> PRT
   <213> Artificial
<220>
   <223> peptide kinase substrate
<400> 88
   Arg Gly Ala Pro Pro Arg Arg Ser Ser Ile Arg Asn Ala
   1 5 10
<210> 89
   <211> 13
   <212> PRT
   <213> Artificial
<220>
   <223> peptide kinase substrate
<400> 89
   Gln Asp Ala Tyr Arg Arg Asn Ser Val Arg Phe Leu Gln
   1 5 10
<210> 90
   <211> 13
   <212> PRT
   <213> Artificial
<220>
   <223> peptide kinase substrate
<400> 90
   Asp Leu Ile Leu Asn Arg Cys Ser Glu Ser Thr Lys Arg
   1 5 10
<210> 91
   <211> 13
   <212> PRT
   <213> Artificial
<220>
   <223> peptide kinase substrate
<400> 91
   Phe Ala Lys Thr Phe Val Gly Thr Pro Tyr Tyr Met Ser
   1 5 10
<210> 92
   <211> 13
   <212> PRT
   <213> Artificial
<220>
   <223> peptide kinase substrate
<400> 92
   Phe Ala Cys Thr Tyr Val Gly Thr Pro Tyr Tyr Val Pro
   1 5 10
<210> 93
   <211> 13
   <212> PRT
   <213> Artificial
<220>
   <223> peptide kinase substrate
<400> 93
   Phe Val Gln Leu Arg Arg Lys Ser Asp Leu Glu Thr Ser
   1 5 10
<210> 94
   <211> 13
   <212> PRT
   <213> Artificial
<220>
   <223> peptide kinase substrate
<400> 94
   Ser Phe Lys Arg Arg Arg Ser Ser Lys Asp Thr Ser Thr
   1 5 10
<210> 95
   <211> 13
   <212> PRT
   <213> Artificial
<220>
   <223> peptide kinase substrate
<400> 95
   Gly Arg Arg Gly Arg Leu Pro Ser Lys Pro Lys Gln Pro
   1 5 10
<210> 96
   <211> 13
   <212> PRT
   <213> Artificial
<220>
   <223> peptide kinase substrate
<400> 96
   Leu His Ala Leu Gly Lys Ala Thr Pro Ile Tyr Leu Asp
   1 5 10
<210> 97
   <211> 13
   <212> PRT
   <213> Artificial
<220>
   <223> peptide kinase substrate
<400> 97
   Glu Pro His Val Thr Arg Arg Thr Pro Asp Tyr Phe Leu
   1 5 10
<210> 98
   <211> 16
   <212> PRT
   <213> Artificial
<220>
   <223> peptide kinase substrate
<400> 98
   Leu Pro Asn Asn Thr Ser Ser Ser Pro Gln Pro Lys Lys Lys Pro Leu
   1 5 10 15
<210> 99
   <211> 13
   <212> PRT
   <213> Artificial
<220>
   <223> peptide kinase substrate
<400> 99
   Gly Thr Pro Thr Arg Lys Ile Ser Ala Ser Glu Phe Asp
   1 5 10
<210> 100
   <211> 13
   <212> PRT
   <213> Artificial
<220>
   <223> peptide kinase substrate
<400> 100
   Gln Glu Lys Arg Arg Gln Ile Ser Ile Arg Gly Ile Val
   1 5 10
<210> 101
   <211> 13
   <212> PRT
   <213> Artificial
<220>
   <223> peptide kinase substrate
<400> 101
   Arg Ser Gly Ser Arg Arg Gly Ser Phe Asp Ala Thr Gly
   1 5 10
<210> 102
   <211> 13
   <212> PRT
   <213> Artificial
<220>
   <223> peptide kinase substrate
<400> 102
   Gly Gln Lys Phe Ala Arg Lys Ser Thr Arg Arg Ser Ile
   1               5                   10
<210> 103
   <211> 13
   <212> PRT
   <213> Artificial
<220>
   <223> peptide kinase substrate
<400> 103
   Glu Glu Gly Thr Phe Arg Ser Ser Ile Arg Arg Leu Ser
   1               5                   10
<210> 104
   <211> 13
   <212> PRT
   <213> Artificial
<220>
   <223> peptide kinase substrate
<400> 104
   Arg Ser Ala Ile Arg Arg Ala Ser Thr Ile Glu Met Pro
   1               5                   10
<210> 105
   <211> 13
   <212> PRT
   <213> Artificial
<220>
   <223> peptide kinase substrate
<400> 105
   Glu Gln Arg Met Lys Glu Ser Ser Phe Tyr Ser Leu Cys
   1               5                   10
<210> 106
   <211> 13
   <212> PRT
   <213> Artificial
<220>
   <223> peptide kinase substrate
<400> 106
   Ala Leu Arg Glu Arg Leu Ser Ser Phe Thr Ser Tyr Glu
   1 5 10
<210> 107
   <211> 13
   <212> PRT
   <213> Artificial
<220>
   <223> peptide kinase substrate
<400> 107
   Phe Met Arg Leu Arg Arg Leu Ser Thr Lys Tyr Arg Thr
   1 5 10
<210> 108
   <211> 15
   <212> PRT
   <213> Artificial
<220>
   <223> peptide kinase substrate
<400> 108
   Gly Leu Arg Arg Trp Ser Leu Gly Gly Leu Arg Arg Trp Ser Leu
   1 5 10 15
<210> 109
   <211> 14
   <212> PRT
   <213> Artificial
<220>
   <223> peptide kinase substrate
<400> 109
   Glu Gly Leu Arg Ser Arg Ser Thr Arg Met Ser Thr Val Ser
   1 5 10
<210> 110
   <211> 13
   <212> PRT
   <213> Artificial
<220>
   <223> peptide kinase substrate
<400> 110
   Lys Ser Asn Val Lys Ile Gln Ser Thr Pro Val Lys Gln
   1 5 10
<210> 111
   <211> 11
   <212> PRT
   <213> Artificial
<220>
   <223> peptide kinase substrate
<400> 111
   Arg Asp Lys Tyr Lys Thr Leu Arg Gln Ile Arg
   1 5 10
<210> 112
   <211> 13
   <212> PRT
   <213> Artificial
<220>
   <223> peptide kinase substrate
<400> 112
   Ser Gln Arg Gln Arg Ser Thr Ser Thr Pro Asn Val His
   1 5 10
<210> 113
   <211> 13
   <212> PRT
   <213> Artificial
<220>
   <223> peptide kinase substrate
<400> 113
   Pro Gly Lys Ala Arg Lys Lys Ser Ser Cys Gln Leu Leu
   1 5 10
<210> 114
   <211> 13
   <212> PRT
   <213> Artificial
<220>
   <223> peptide kinase substrate
<400> 114
   Ala Val Ile Pro Ile Asn Gly Ser Pro Arg Thr Pro Arg
   1 5 10
<210> 115
   <211> 13
   <212> PRT
   <213> Artificial
<220>
   <223> peptide kinase substrate
<400> 115
   Ile Tyr Ile Ser Pro Leu Lys Ser Pro Tyr Lys Ile Ser
   1               5                   10
<210> 116
   <211> 13
   <212> PRT
   <213> Artificial
<220>
   <223> peptide kinase substrate
<400> 116
   Lys Glu Asn Ser Pro Cys Val Thr Pro Val Ser Thr Ala
   1               5                   10
<210> 117
   <211> 13
   <212> PRT
   <213> Artificial
<220>
   <223> peptide kinase substrate
<400> 117
   Asp Glu Ile Cys Ile Ala Gly Ser Pro Leu Thr Pro Arg
   1               5                   10
<210> 118
   <211> 13
   <212> PRT
   <213> Artificial
<220>
   <223> peptide kinase substrate
<400> 118
   Cys Ile Ala Gly Ser Pro Leu Thr Pro Arg Arg Val Thr
   1               5                   10
<210> 119
   <211> 13
   <212> PRT
   <213> Artificial
<220>
   <223> peptide kinase substrate
<400> 119
   Gly Leu Gly Arg Ser Ile Thr Ser Pro Thr Thr Leu Tyr
   1               5                   10
<210> 120
   <211> 13
   <212> PRT
   <213> Artificial
<220>
   <223> peptide kinase substrate
<400> 120
   Asp Ser Pro Ser Asp Gly Gly Thr Pro Gly Arg Met Pro
   1               5                   10
<210> 121
   <211> 13
   <212> PRT
   <213> Artificial
<220>
   <223> peptide kinase substrate
<400> 121
   Glu Leu Asn Lys Asp Arg Thr Ser Arg Asp Ser Ser Pro
   1               5                   10
<210> 122
   <211> 13
   <212> PRT
   <213> Artificial
<220>
   <223> peptide kinase substrate
<400> 122
   Asp Arg Thr Ser Arg Asp Ser Ser Pro Val Met Arg Ser
   1               5                   10
<210> 123
   <211> 13
   <212> PRT
   <213> Artificial
<220>
   <223> peptide kinase substrate
<400> 123
   Lys Met Gln Leu Arg Arg Pro Ser Asp Gln Glu Val Ser
   1               5                   10
<210> 124
   <211> 13
   <212> PRT
   <213> Artificial
<220>
   <223> peptide kinase substrate
<400> 124
   Ile Ala Lys Arg Arg Arg Leu Ser Ser Leu Arg Ala Ser
   1 5 10
<210> 125
   <211> 13
   <212> PRT
   <213> Artificial
<220>
   <223> peptide kinase substrate
<400> 125
   Val Arg Arg Leu Arg Arg Leu Thr Ala Arg Glu Ala Ala
   1 5 10
<210> 126
   <211> 13
   <212> PRT
   <213> Artificial
<220>
   <223> peptide kinase substrate
<400> 126
   Lys Asn Gly Cys Arg Arg Gly Ser Ser Leu Gly Gln Ile
   1 5 10
<210> 127
   <211> 13
   <212> PRT
   <213> Artificial
<220>
   <223> peptide kinase substrate
<400> 127
   Leu Ile Glu Asp Asn Glu Tyr Thr Ala Arg Gln Gly Ala
   1 5 10
<210> 128
   <211> 13
   <212> PRT
   <213> Artificial
<220>
   <223> peptide kinase substrate
<400> 128
   Lys Thr Pro Ser Ser Pro Val Tyr Gln Asp Ala Val Ser
   1 5 10
<210> 129
   <211> 13
   <212> PRT
   <213> Artificial
<220>
   <223> peptide kinase substrate
<400> 129
   Ser Ser Arg Arg Thr Thr Leu Cys Gly Thr Leu Asp Tyr
   1 5 10
<210> 130
   <211> 13
   <212> PRT
   <213> Artificial
<220>
   <223> peptide kinase substrate
<400> 130
   Ala Ala Gly Glu Arg Arg Lys Ser Gln Glu Ala Gln Val
   1 5 10
<210> 131
   <211> 13
   <212> PRT
   <213> Artificial
<220>
   <223> peptide kinase substrate
<400> 131
   Gly Ser Arg Ser Arg Thr Pro Ser Leu Pro Thr Pro Pro
   1 5 10
<210> 132
   <211> 13
   <212> PRT
   <213> Artificial
<220>
   <223> peptide kinase substrate
<400> 132
   Glu Pro Pro Ser Pro Ala Thr Thr Pro Cys Gly Lys Val
   1 5 10
<210> 133
   <211> 13
   <212> PRT
   <213> Artificial
<220>
   <223> peptide kinase substrate
<400> 133
   His Gln Arg Arg Lys Tyr Arg Ser Asn Lys Gly Glu Ser
   1               5                   10
<210> 134
   <211> 15
   <212> PRT
   <213> Artificial
<220>
   <223> peptide kinase substrate
<400> 134
   Arg Phe Ile Gly Arg Arg Gln Ser Leu Ile Glu Asp Ala Arg Lys
   1               5                   10                  15
<210> 135
   <211> 13
   <212> PRT
   <213> Artificial
<220>
   <223> peptide kinase substrate
<400> 135
   Glu His Ile Glu Arg Arg Val Ser Asn Ala Gly Gly Pro
   1               5                   10
<210> 136
   <211> 13
   <212> PRT
   <213> Artificial
<220>
   <223> peptide kinase substrate
<400> 136
   Gly Ala Lys Leu Arg Lys Val Ser Lys Gln Glu Glu Ala
   1               5                   10
<210> 137
   <211> 13
   <212> PRT
   <213> Artificial
<220>
   <223> peptide kinase substrate
<400> 137
   Leu Ala Arg Arg Arg Lys Ala Thr Gln Val Gly Glu Lys
   1               5                   10
<210> 138
   <211> 13
   <212> PRT
   <213> Artificial
<220>
   <223> peptide kinase substrate
<400> 138
   Glu Glu Lys Lys Lys Lys Thr Thr Thr Ile Ala Val Glu
   1               5                   10
<210> 139
   <211> 13
   <212> PRT
   <213> Artificial
<220>
   <223> peptide kinase substrate
<400> 139
   Asn Gln Asn Ser Arg Arg Pro Ser Arg Ala Thr Trp Leu
   1               5                   10
<210> 140
   <211> 13
   <212> PRT
   <213> Artificial
<220>
   <223> peptide kinase substrate
<400> 140
   Leu Gly Ala Asp Asp Ser Tyr Tyr Thr Ala Arg Ser Ala
   1               5                   10